# EUROPEAN PATENT APPLICATION

(11) **EP 4 273 269 A1**
(43) Date of publication of application: **08.11.2023**
(21) Application number: 21913421.0
(22) Date of filing: 29.10.2021
(51) Int. Cl.: C12Q 1/6886, C12N 15/11, A61P 35/00, A61K 45/00

(54) **TUMOR MARKER AND APPLICATION THEREOF**

(30) Priority: 30.12.2020 CN 202011608940
(71) Applicant: Shanghai Epiprobe Biotechnology Co., Ltd., Shanghai 200233 (CN)
(72) Inventor: DONG, Shihua, Shanghai 200233 (CN); LI, Zhenyan, Shanghai 200233 (CN)
(74) Representative: IPLodge bv
(86) International application number: PCT/CN2021/127464
(87) International publication number: WO 2022/142677

(57) **Abstract**

The present invention provides a tumor marker and an application thereof. The tumor marker is located on the chromosome 1, chromosome 2, chromosome 3, chromosome 5, chromosome 7, chromosome 8, chromosome 10, chromosome 11, and chromosome 21 of the human genome, and comprises one or more CpG sites capable of undergoing methylation modification. The present invention provides a class of DNA epigenetic modification-related tumor markers that exhibit a significant high methylation status in tumor patients. The tumor markers can be used for clinical assisted screening, diagnosis, prognosis and the like of tumors, or can be used for designing diagnostic reagents and kits.

## Description

### Technical Field

The disclosure relates to the field of biotechnology, in particular, the disclosure relates to a tumor marker and application thereof.

### Background

Tumorigenesis is a complex, multi-level and multi-factor dynamic process, comprising numerous interacting factors such as external environmental factors, genetic variation, and epigenetic changes. External environmental factors comprise physical, chemical, biological and other carcinogenic factors and unhealthy living habits; genetic variation comprises gene mutation, copy number change, chromosomal dislocation, etc.; epigenetic changes mainly comprise DNA methylation, histone modification, non-coding RNA and other variable factors. In the process of tumorigenesis, above factors are complementary and collectively-effective, leading to a series of inactivation of tumor suppressor genes and activation of proto-oncogenes, then resulting in tumors. Timely detection and diagnosis are of great significance in tumor treatment.

With deepening understanding of tumors and advances in science and technology, many novel tumor markers have been discovered and used in clinical diagnosis. Before 1980, tumor markers were mainly cellular secretions including hormones, enzymes, proteins etc., such as carcinoembryonic antigen (CEA), alpha-fetoprotein (AFP), etc., using as markers for gastric cancer, liver hepatocellular carcinoma and other various tumors. Recently, although this type of tumor markers is still used in clinical, its sensitivity and accuracy cannot meet current clinical needs. Now there are more and more markers based on mutations, such as tumor suppressor p53 mutation, BRCA gene mutation, microsatellite instability in colorectal cancer, and so on. However, such markers are not ideal in early tumor discovery and diagnosis. DNA methylation-based markers allow tumors to be diagnosed in early stage of tumor development.

Epigenomics is a subject of studying heritable changes in gene functions without DNA sequence changes and ultimately leading to phenotypic changes. mainly comprise DNA methylation, histone modification, microRNA level changes and other biochemical processes. DNA methylation is a well-researched epigenetic mechanism, with application prospects in tumor clinical practice including diagnosis and treatment. DNA methylation refers to a process of transferring methyl groups to specific bases using S-adenosylmethionine (SAM) as a methyl donor under the catalysis of DNA methyltransferase (DMT) in vivo. In mammals, DNA methylation mainly occurs at the C of 5'-CpG-3' to generate 5-methylcytosine (5mC).

In normal cells, some CpGs are in status of hypermethylated/transcriptionally silenced, while in tumor cells these CpGs are extensively demethylated, resulting in transcription of repetitive sequences, activation of transposons, highly-unstable genome and enhanced transcription of proto-oncogenes. In addition, some CpG islands that were originally hypomethylated in normal cells were hypermethylated in tumor cells, resulting in transcriptional inactivation of genes, including DNA repair genes, cell cycle genes and anti-apoptotic genes, etc. After the genomic DNA is treated with bisulfite, the methylation status of specific sites can be effectively determined by PCR or sequencing. Using the techniques described above, these abnormal DNA methylation statuses can be detected at an early stage of tumorigenesis.

Although complete sequences of the human genome have been mastered by people, the genome sequences are complex, and it is still unclear that which genes or which segments are closely related to diseases. In addition, due to the complexity of tumor itself, it is more difficult to find sensitive and specific tumor markers. In the present disclosure, by using a large amount of data combined with optimized methods, the inventors found tumor markers with improved accuracy and provided more ways for tumor diagnosis.

### Summary of the disclosure

To overcome the defects of the prior art, the disclosure provides a class of tumor markers related to DNA epigenetic modification, and the markers show a significant hypermethylation status in patients with tumor.

In the first aspect, the present disclosure provides a tumor marker, the tumor marker is located on the chromosome 1, chromosome 2, chromosome 3, chromosome 5, chromosome 7, chromosome 8, chromosome 10, chromosome 11, and chromosome 21 of the human genome, and comprises one or more CpG sites can be subject to methylation modification.

Preferably, the methylation modification comprises 5-formylcytosine(5fC) modification, 5-hydroxymethylcytosine(5hmC) modification, 5-methylcytosine(5mC) modification or 5-carboxylcytosine(5-caC) modification.

Preferably, the tumor marker comprises one or more regions corresponding to the human genome Hg19 (coordinates start from 0) located at
chr2:105459135-105459190, chr10:124902392-124902455,
chr3:157812331-157812498, chr21:38378275-38378539,
chr8:97170353-97170404, chr5:134880362-134880455,
chr10:94835119-94835252, chr11:31826557-31826963,
chr3:147114032-147114108, chr10:50819227-50819589,
chr2:66809255-66809281, chr7:97361393-97361461,
chr8:70984200-70984294, chr1:6515341-6515409.

Preferably, the sequence of chr2:105459135-105459190 region is selected from the following group:
a) a base sequence as shown in SEQ ID NO.1;
b) a complementary sequence of the base sequence as shown in SEQ ID NO.1;
c) a nucleotide sequence or a complementary sequence thereof with at least 70% homology to SEQ ID NO.1.
and/or the sequence of chr10: 124902392-124902455 region is selected from the following group:
a) a base sequence as shown in SEQ ID NO.2;
b) a complementary sequence of the base sequence as shown in SEQ ID NO.2;
c) a nucleotide sequence or a complementary sequence thereof with at least 70% homology to SEQ ID NO.2.
and/or the sequence of chr3:157812331-157812498 region is selected from the following group:
a) a base sequence as shown in SEQ ID NO.3;
b) a complementary sequence of the base sequence as shown in SEQ ID NO.3;
c) a nucleotide sequence or a complementary sequence thereof with at least 70% homology to SEQ ID NO.3.
and/or the sequence of chr21:38378275-38378539 region is selected from the following group:
a) a base sequence as shown in SEQ ID NO.4;
b) a complementary sequence of the base sequence as shown in SEQ ID NO.4;
c) a nucleotide sequence or a complementary sequence thereof with at least 70% homology to SEQ ID NO.4.
and/or the sequence of chr8:97170353-97170404 region is selected from the following group:
a) a base sequence as shown in SEQ ID NO.5;
b) a complementary sequence of the base sequence as shown in SEQ ID NO.5;
c) a nucleotide sequence or a complementary sequence thereof with at least 70% homology to SEQ ID NO.5.
and/or the sequence of chr5:134880362-134880455 region is selected from the following group:
a) a base sequence as shown in SEQ ID NO.6;
b) a complementary sequence of the base sequence as shown in SEQ ID NO.6;
c) a nucleotide sequence or a complementary sequence thereof with at least 70% homology to SEQ ID NO.6.
and/or the sequence of chr10:94835119-94835252 region is selected from the following group:
a) a base sequence as shown in SEQ ID NO.7;
b) a complementary sequence of the base sequence as shown in SEQ ID NO.7;
c) a nucleotide sequence or a complementary sequence thereof with at least 70% homology to SEQ ID NO.7.
and/or the sequence of chr11:31826557-31826963 region is selected from the following group:
a) a base sequence as shown in SEQ ID NO.8;
b) a complementary sequence of the base sequence as shown in SEQ ID NO.8;
c) a nucleotide sequence or a complementary sequence thereof with at least 70% homology to SEQ ID NO.8.
and/or the sequence of chr3:147114032-147114108 region is selected from the following group:
a) a base sequence as shown in SEQ ID NO.9;
b) a complementary sequence of the base sequence as shown in SEQ ID NO.9;
c) a nucleotide sequence or a complementary sequence thereof with at least 70% homology to SEQ ID NO.9.
and/or the sequence of chr10:50819227-50819589 region is selected from the following group:
a) a base sequence as shown in SEQ ID NO.10;
b) a complementary sequence of the base sequence as shown in SEQ ID NO.10;
c) a nucleotide sequence or a complementary sequence thereof with at least 70% homology to SEQ ID NO.10.
and/or the sequence of chr2:66809255-66809281 region is selected from the following group:
a) a base sequence as shown in SEQ ID NO.11;
b) a complementary sequence of the base sequence as shown in SEQ ID NO.11;
c) a nucleotide sequence or a complementary sequence thereof with at least 70% homology to SEQ ID NO.11.
and/or the sequence of chr7:97361393-97361461 region is selected from the following group:
a) a base sequence as shown in SEQ ID NO.12;
b) a complementary sequence of the base sequence as shown in SEQ ID NO.12;
c) a nucleotide sequence or a complementary sequence thereof with at least 70% homology to SEQ ID NO.12.
and/or the sequence of chr8:70984200-70984294 region is selected from the following group:
a) a base sequence as shown in SEQ ID NO.13;
b) a complementary sequence of the base sequence as shown in SEQ ID NO.13;
c) a nucleotide sequence or a complementary sequence thereof with at least 70% homology to SEQ ID NO.13.
and/or the sequence of chr1:6515341-6515409 region is selected from the following group:
a) a base sequence as shown in SEQ ID NO.14;
b) a complementary sequence of the base sequence as shown in SEQ ID NO.14;
c) a nucleotide sequence or a complementary sequence thereof with at least 70% homology to SEQ ID NO.14.

The above sequences are shown in Table 1 below, and the complementary sequences thereof are shown in Table 2. Wherein, bold italic font represents the CpG site, and the number under heavy symbol represents the number of detection site.

**Table 1**

| Coordinates on Hg19 | Sequences | Serial number |
|---|---|---|
| chr2:105459135-105459190 | GCACTCACACGTACACCCGGTCCTCGCACGCGCACACACG 01 02 03 04 05 06 CACACTGTTCCCCGCC 07 08 | SEQ ID NO.1 |
| chr10: 124902392-124902455 | CGCTAGCGGTCCAAATGTAGACTCCGCAAAGAGGCTAAGAA 01 02 03 GACCTGTCTACCCGGGTGCGCGC 04 05 06 07 | SEQ ID NO.2 |
| chr3:157812331-157812498 | GCCGAACCCACCTGGCTCCTATCGCACGGGACATTCCCGAC 01 02 03 04 CCACCCACGCCGCGTCACTGAGCCTCTGTACCGATACCCG 05 06 07 08 09 GCGCCTCCGCCAGCAGGGCCTGGACGCACCGCCTCCTTTG 10 11 12 13 ACCTCGGGCTTCCCCCGCGCTCCGCTGCTTGGGGCAGACT 14 15 16 17 GGCCCCG 18 | SEQ ID NO.3 |
| chr21:38378275-38378539 | CGGGTTCGAGCCCTGGCGTCGGGCGTCCGGGAGCCCA 01 02 03 04 05 06 CTGTCCAGCACCGAAGGCAAGGCCGGTGCACGCGGACCC 07 08 09 10 11 GAGGATTCGGTAGATGTCCCCGAAGACCCGCTGCCGCTCTA 12 13 14 15 AGGCGGTGGAAGCGAGATTCTCCGGAAACCCAGGGAATCC 16 17 18 19 GATGCTCGCACAGGACCAAAGCCCGAGGCCGCGGGGACC 20 21 22 23 ACAGAGGGACGGAGAAGCCGGGACTCCTCACATCCCACATC 24 25 CGGCAGGGGAAGCCCAGCAGGTGAGCG 26 27 | SEQ ID NO.4 |
| chr8:97170353-97170404 | CGTCTCCTCCCGCTGTGGACCGCTCGCATCCCCAGCCCTC 01 02 03 04 CACCGCATTCCG 05 06 | SEQ ID NO.5 |
| chr5:134880362-134880455 | CGCCGCAATCTGTCGGGACCCGCCGGGTTTCCATATGAAG 01 02 03 04 05 GGTCGAGCCGGCGCCTTGGGAGCGCTGAATGGCCGCTCG 06 07 08 09 10 11 CGGTCCGGCGGGCGC 12 13 14 15 16 | SEQ ID NO.6 |
| chr10:94835119-9483 5252 | CGCTGTTCCTGGGGCCCCCAAAGCGCGCGCCTGGGGCCC 01                                    02 03 04 AGCTTTCTGGAGTGGGCGGCCGGCTCAGACTACAGCTATGG 05    06 AATCCCGAAGGAAGGCTGAGACACCCGGTCAGGAGAGCTG 07                  08 CGGAAGGGGCTGCG 09          10 | SEQ ID NO.7 |
| chr11:31826557-31826963 | CGCTCTCGCCCACTGGCGATGATTATGCGCCTAGAACTCGA 01 02 03 04 05 CCGCGAAGCAACTAATAGGAAAACATATGGTGTCAATTTGGAT 06 07 GCTCCGCGCCTCGCGCACACCGGGAACGAGCGGCACA 08 09 10 11 12 13 14 AAGCCCTGCCGGCCGGCCCGCGACCCCGCGCCCCTCGG 15 16 17 18 19 20 21 GGCCTGCCAGCCGGGCCGCAGCGACAAACGCTCAGGGCT 22 23 24 25 GCGCGCCCTGGCTGGGGCCCGCCCGAGAGACAGCCTGCG 26 27 28 29 30 GCTGGGGAGTCTGAGCTCCAAGGGGAGAGCCCAGCCGCCG 31 32 AAGGCGAGCCTACCGGCCAAGCCCTGGGGTCCGGCAGGTT 33 34 35 CTGCACAACTACTCCCGCAAAGCTCGCCACCTTTGTGCCCTT 36 37 TCCTCAGCTACGCGCTTACCAGCCCCGGAAGCACCAGGGG 38 39 40 GCGACCG 41 42 | SEQ ID NO.8 |
| chr3:147114032-147114108 | GTAGCCATGCAGGGCTGCGGCAGCTGCCAGGGCGTCGCTG 01 02 03 CGGGCCGCAGGCCCTGGCGGGAAGGGCTCCGGCCGCG 04 05 06 07 08 09 | SEQ ID NO.9 |
| chr10:50819227-50819589 | CGCATGAGCTACGACGTGCCGCTGCTGATCGGCCTGGGC 01          02  03    04          05          06 GTCATGTTCGCCTCTACAGTCCTGTTCGCCTTCGCCGAGGA 07                  08      09  10 CTACGCCACGCTGTTCGCGGCGCGCAGCCTGCAGGGCCT 11    12      13 14 15 16 GGGCTCAGCCTTCGCCGACACGTCTGGCATAGCCATGATCG 17  18    19                  20 CCGATAAGTACCCGGAGGAGCCGGAGCGCAGTCGTGCACT 21          22        23    24     25 GGGCGTGGCGCTGGCCTTCATTAGCTTCGGAAGCCTAGTGG 26    27                28 CCCCGCCCTTCGGGGGCATCCTCTATGAGTTCGCCGGCAA 29      30                    31  32 GCGCGTGCCCTTCTTGGTGCTAGCTGCCGTGTCGCTCTTTG 33 34                      35    36 ACGCGCTGTTGCTGCTGGCAGTGGCCAAACCCTTCTCGGC 37 38                                39 40 G | SEQ ID NO. 10 |
| chr2:66809255-66809281 | GCGCGCGCCCAAGGGCGTGCCCACCGC .  .  .  .  .  . 01 02 03 04 05 06 | SEQ ID NO. 11 |
| chr7:97361393-97361461 | CGAAAGAGCGCGCTCGGACCTCCTTCCCGGCGGCAGCTA 01 02 03 04 05 06 CCGAGAGTGCGGAGCGACCAGCGTGCGCTC 07 08 09 10 11 12 | SEQ ID NO. 12 |
| chr8:70984200-70984294 | CGCAGGCTCTGAAAACCAAGGTCCAGACCCATACCGAGCGC 01 02 03 CCAAAGACCGCATTTGAATTCTCCTCGCCGGCCATTGAGGG 04 05 06 AGAGAAAGGAACG 07 | SEQ ID NO. 13 |
| chr1:6515341-6515409 | CGTTTACCTCTTCCTGGAGCACTGGCGCCGCTGGGCCTGC 01                                    02   03 CGCGGACCAGGCCGCCGCGCCCAGGCGCG 04   05              06    07   08          09   10 | SEQ ID NO. 14 |

**Table 2**

| Coordinates on Hg 19 | Sequences | Serial number |
|---|---|---|
| chr2:105459135-105459190 | GGCGGGGAACAGTGTGCGTGTGTGCGCGTGCGAGGACCG 01 02 03 04 05 06 GGTGTACGTGTGAGTGC 07 08 | SEQ ID NO.15 |
| chr10:124902392-124902455 | GCGCGCACCCGGGTAGACAGGTCTTCTTAGCCTCTTTGCGG 01 02 03 04 AGTCTACATTTGGACCGCTAGCG 05 06 | SEQ ID NO.16 |
| chr3:157812331-157812498 | CGGGGCCAGTCTGCCCCAAGCAGCGGAGCGCGGGGGAAG 01 02 03 04 CCCGAGGTCAAAGGAGGCGGTGCGTCCAGGCCCTGCTGGC 05 06 07 08 GGAGGCGCCGGGTATCGGTACAGAGGCTCAGTGACGCGG 09 10 11 12 13 CGTGGGTGGTCGGGAATGTCCCGTGCGATAGGAGCCAGG 14 15 16 17 TGGGTTCGGC 18 19 | SEQ ID NO.17 |
| chr21:38378275-38378539 | CGCTCACCTGCTGGGCTTCCCCTGCCGGATGTGGGATGTGA 01                                        02 GGAGTCCCGGCTTCTCCGTCCCTCTGTGGTCCCCGCGGCC 03        04                    05 06 TCGGGCTTTGGTCCTGTGCGAGCATCGGATTCCCTGGGTTT 07                08        09 CCGGAGAATCTCGCTTCCACCGCCTTAGAGCGGCAGCGGG 10          11          12          13      14 TCTTCGGGGACATCTACCGAATCCTCGGGTCCGCGTGCAC 15              16        17      18 19 CGGCCTTGCCTTCGGTGCTGGACAGTGGGCTCCCGGACGC 20              21                        22    23 CCCGACGCCAGGGCTCGAACCCG 24  25          26        27 | SEQ ID NO.18 |
| chr8:97170353-97170404 | CGGAATGCGGTGGAGGGCTGGGATGCGAGCGGTCCACA 01 02 03 04 GCGGGAGGAGACG 05 06 | SEQ ID NO.19 |
| chr5:134880362-134880455 | GCGCCCGCCGGACCGCGAGCGGCCATTCAGCGCTCCCAA 01 02 03 04 05 06 07 GGCGCCGGCTCGACCCTTCATATGGAAACCCGGCGGGTCC 08 09 10 11 12 CGACAGATTGCGGCG 14 15 16 | SEQ ID NO.20 |
| chr10:94835119-9483 5252 | CGCAGCCCCTTCCGCAGCTCTCCTGACCGGGTGTCTCAGC 01 02 03 CTTCCTTCGGGATTCCATAGCTGTAGTCTGAGCCGGCCGCC 04 05 06 CACTCCAGAAAGCTGGGCCCCAGGCGCGCGCTTTGGGGGC 07 08 09 CCCAGGAACAGCG 10 | SEQ ID NO.21 |
| chr11:31826557-31826963 | CGGTCGCCCCCTGGTGCTTCCGGGGCTGGTAAGCGCGTAG 01 02 03 04 05 CTGAGGAAAGGGCACAAAGGTGGCGAGCTTTGCGGGAGTA 06 07 GTTGTGCAGAACCTGCCGGACCCCAGGGCTTGGCCGGTAG 08 09 GCTCGCCTTCGGCGGCTGGGCTCTCCCCTTGGAGCTCAGA 10 11 12 CTCCCCAGCCGCAGGCTGTCTCTCGGGCGGGCCCCAGCCA 13 14 15 GGGCGCGCAGCCCTGAGCGTTTGTCGCTGCGGCCCGGCT 16 17 18 19 20 21 GGCAGGCCCCGAGGGGCGCGGGGTCGCGGGCCGGCCG 22 23 24 25 26 27 28 GCAGGGCTTTGTGCCGCTCGTTCCCGGGTGTGCGCGAGG 29 30 31 32 33 CGCGGAGCATCCAAATTGACACCATATGTTTTCCTATTAGTTG 34 35 CTTCGCGGTCGAGTTCTAGGCGCATAATCATCGCCAGTGGG 36 37 38 39 40 CGAGAGCG 41 42 | SEQ ID NO.22 |
| chr3:147114032-147114108 | CGCGGCCGGAGCCCTTCCCGCCAGGGCCTGCGGCCCGC 01 02 03 04 05 06 AGCGACGCCCTGGCAGCTGCCGCAGCCCTGCATGGCTAC 07 08 09 10 | SEQ ID NO.23 |
| chr10:50819227-50819589 | CGCCGAGAAGGGTTTGGCCACTGCCAGCAGCAACAGCGCG 01 02 03 04 TCAAAGAGCGACACGGCAGCTAGCACCAAGAAGGGCACGC 05 06 07 08 GCTTGCCGGCGAACTCATAGAGGATGCCCCCGAAGGGCGG 09 10 11 12 GGCCACTAGGCTTCCGAAGCTAATGAAGGCCAGCGCCACGC 13 14 15 CCAGTGCACGACTGCGCTCCGGCTCCTCCGGGTACTTATC 16 17 18 19 20 GGCGATCATGGCTATGCCAGACGTGTCGGCGAAGGCTGAG 21 22 23 24 CCCAGGCCCTGCAGGCTGCGCGCCGCGAACAGCGTGGC 25 26 27 28 29 30 GTAGTCCTCGGCGAAGGCGAACAGGACTGTAGAGGCGAAC 31 32 33 34 ATGACGCCCAGGCCGATCAGCAGCGGCACGTCGTAGCTCA 35 36 37 38 39 TGCG 40 | SEQ ID NO.24 |
| chr2:66809255-66809281 | GCGGTGGGCACGCCCTTGGGCGCGCGC 01 02 03 04 05 | SEQ ID NO.25 |
| chr7:97361393-97361461 | GAGCGCACGCTGGTCGCTCCGCACTCTCGGTAGCTGCCGC 01 02 03 04 05 CGGGAAGGAGGTCCGAGCGCGCTCTTTCG 06 07 08 09 10 | SEQ ID NO.26 |
| chr8:70984200-70984294 | CGTTCCTTTCTCTCCCTCAATGGCCGGCGAGGAGAATTCAAA 01 02 03 TGCGGTCTTTGGGCGCTCGGTATGGGTCTGGACCTTGGTTT 04 05 06 TCAGAGCCTGCG 07 | SEQ ID NO.27 |
| chr1:6515341-6515409 | CGCGCCTGGGCGCGGCGGCCTGGTCCGCGGCAGGCCCA 01 02 03 04 05 06 07 GCGGCGCCAGTGCTCCAGGAAGAGGTAAACG 08 09 10 | SEQ ID NO.28 |

In the second aspect, the present disclosure provides a use of the tumor marker of the present disclosure in tumor screening, prognosis, diagnostic reagents and drug targets.

Preferably, the use comprises detecting the methylation statuses of CpG sites of the tumor markers; further preferably, the CpG sites are shown in Table 1 and Table 2.

Preferably, the tumor comprises: bladder adenocarcinoma(BLCA), cervical cancer(CESC), cholangiocarcinoma(CHOL), colon adenocarcinoma(COADREAD), esophageal cancer(ESCA), glioblastoma(GBM), head and neck cancer(HNSC), kidney renal clear cell carcinoma(KIRC), kidney renal papillary cell carcinoma(KIRP), liver hepatocellular carcinoma(LIHC), lung adenocarcinoma(LUNG), pancreatic adenocarcinoma(PAAD), pheochromocytoma and paraganglioma(PCPG), prostate adenocarcinoma(PRAD), sarcoma (SARC), skin cutaneous melanoma(SKCM), stomach adenocarcinoma(STAD), thyroid carcinoma(THCA), thymoma(THYM), uterine corpus endometrial carcinoma(UCEC), breast cancer(BC); more preferably, the tumor comprises: breast cancer(BC), cervical carcinoma(CESC), esophageal cancer(ESCA), head and neck cancer(HNSC), lung adenocarcinoma(LUNG), pancreatic adenocarcinoma(PAAD).

Preferably, tumors for which SEQ ID NO.1 or SEQ ID NO.15 can be used as markers comprise: BLCA, CESC, CHOL, COADREAD, ESCA, GBM, HNSC, KIRC, KIRP, LIHC, LUNG, PAAD, PCPG, PRAD, SARC, SKCM, STAD, THCA, THYM, UCEC; more preferably, tumors for which SEQ ID NO.1 or SEQ ID NO.15 can be used as markers comprise: BLCA, CESC, CHOL, COADREAD, ESCA, GBM, HNSC, KIRC, KIRP, LUNG, PCPG, PRAD, SKCM, STAD, THCA, THYM, UCEC.

Preferably, tumors for which SEQ ID NO.2 or SEQ ID NO.16 can be used as markers comprise: BC, CESC, ESCA, HNSC, LUNG, PAAD.

Preferably, tumors for which SEQ ID NO.3 or SEQ ID NO.17 can be used as markers comprise: BLCA, CESC, CHOL, COADREAD, ESCA, GBM, HNSC, KIRC, KIRP, LIHC, LUNG, PAAD, PCPG, PRAD, SARC, SKCM, STAD, THCA, THYM, UCEC.

Preferably, tumors for which SEQ ID NO.4 or SEQ ID NO.18 can be used as markers comprise: BLCA, CESC, CHOL, COADREAD, ESCA, GBM, HNSC, KIRC, KIRP, LIHC, LUNG, PAAD, PCPG, PRAD, SARC, SKCM, STAD, THCA, THYM, UCEC; more preferably, tumors for which SEQ ID NO.4 or SEQ ID NO.18 can be used as markers comprise: BLCA, CESC, CHOL, COADREAD, ESCA, GBM, HNSC, KIRC, KIRP, LIHC, LUNG, PAAD, PRAD, SARC, SKCM, STAD, THCA, THYM, UCEC.

Preferably, tumors for which SEQ ID NO.5 or SEQ ID NO.19 can be used as markers comprise: BLCA, CESC, CHOL, COADREAD, ESCA, GBM, HNSC, KIRC, KIRP, LIHC, LUNG, PAAD, PCPG, PRAD, SARC, SKCM, STAD, THCA, THYM, UCEC; more preferably, tumors for which SEQ ID NO.5 or SEQ ID NO.19 can be used as markers comprise: BLCA, CESC, CHOL, COADREAD, ESCA, GBM, HNSC, KIRC, KIRP, LIHC, LUNG, PAAD, PRAD, SARC, SKCM, STAD, THCA, THYM, UCEC.

Preferably, tumors for which SEQ ID NO.6 or SEQ ID NO.20 can be used as markers comprise: BLCA, CESC, CHOL, COADREAD, ESCA, GBM, HNSC, KIRC, KIRP, LIHC, LUNG, PAAD, PCPG, PRAD, SARC, SKCM, STAD, THCA, THYM, UCEC; more preferably, tumors for which SEQ ID NO.6 or SEQ ID NO.20 can be used as markers comprise: BLCA, CESC, CHOL, COADREAD, ESCA, HNSC, KIRC, KIRP, LIHC, LUNG, PAAD, PRAD, SARC, SKCM, STAD, THCA, THYM, UCEC.

Preferably, tumors for which SEQ ID NO.7 or SEQ ID NO.21 can be used as markers comprise: BC, CESC, ESCA, HNSC, LUNG, PAAD.

Preferably, tumors for which SEQ ID NO.8 or SEQ ID NO.22 can be used as markers comprise: BLCA, CESC, CHOL, COADREAD, ESCA, GBM, HNSC, KIRC, KIRP, LIHC, LUNG, PAAD, PCPG, PRAD, SARC, SKCM, STAD, THCA, THYM, UCEC; more preferably, tumors for which SEQ ID NO.8 or SEQ ID NO.22 can be used as markers comprise: BLCA, CESC, CHOL, COADREAD, ESCA, GBM, HNSC, KIRC, KIRP, LIHC, LUNG, PAAD, PRAD, SARC, SKCM, STAD, THCA, THYM, UCEC.

Preferably, tumors for which SEQ ID NO.9 or SEQ ID NO.23 can be used as markers comprise: BC, CESC, ESCA, HNSC, LUNG, PAAD.

Preferably, tumors for which SEQ ID NO.10 or SEQ ID NO.24 can be used as markers comprise: BLCA, CESC, CHOL, COADREAD, ESCA, GBM, HNSC, KIRC, KIRP, LIHC, LUNG, PAAD, PCPG, PRAD, SARC, SKCM, STAD, THCA, THYM, UCEC.

Preferably, tumors for which SEQ ID NO.11 or SEQ ID NO.25 can be used as markers comprise: BC, CESC, ESCA, HNSC, LUNG, PAAD.

Preferably, tumors for which SEQ ID NO. 12 or SEQ ID NO.26 can be used as markers comprise: BLCA, CESC, CHOL, COADREAD, ESCA, GBM, HNSC, KIRC, KIRP, LIHC, LUNG, PAAD, PCPG, PRAD, SARC, SKCM, STAD, THCA, THYM, UCEC.

Preferably, tumors for which SEQ ID NO. 13 or SEQ ID NO.27 can be used as markers comprise: BLCA, CESC, CHOL, COADREAD, ESCA, GBM, HNSC, KIRC, KIRP, LIHC, LUNG, PAAD, PCPG, PRAD, SARC, SKCM, STAD, THCA, THYM, UCEC.

Preferably, tumors for which SEQ ID NO. 14 or SEQ ID NO.28 can be used as markers comprise: BC, CESC, ESCA, HNSC, LUNG, PAAD.

In the third aspect, the present disclosure provides a drug used for inhibiting tumor proliferation, wherein the drug comprises an inhibitor of tumor markers according to the present disclosure.

Preferably, the inhibitor is a methylation inhibitor; more preferably, the inhibitor is an inhibitor that inhibit the methylation of one or more CpG sites in Table 1 and Table 2.

Preferably, the drug also comprises a pharmaceutically acceptable carrier.

In the fourth aspect, the present disclosure provides a method for detecting tumor markers, comprising the following steps:
S1. Obtaining a tissue sample to be tested;
S2. Extracting DNA of the tissue sample to be tested, and obtaining a methylation value of the sample;
S3. Calculating methylation status of each CpG site in the tumor marker sequence region or the average methylation status of the entire region.

Preferably, the method for obtaining the methylation value of the sample in step S2 comprises: sequencing, probes detecting, antibodies detecting, and mass spectrometry detecting.

In the fifth aspect, the present disclosure provides a kit for detecting tumor markers, comprising primers or probes for specifically detecting the tumor markers of the present disclosure.

Preferably, said specifically detecting of the tumor markers comprises specifically detecting of the methylation of CpG sites of the tumor markers.

Preferably, the methylation of CpG sites of tumor markers comprises 5-formylcytosine(5fC) modification, 5-hydroxymethylcytosine(5hmC) modification, 5-methylcytosine(5mC) modification or 5-carboxylcytosine(5-caC) modification.

Compared with the prior art, the present disclosure has the following advantages: the disclosure provides a class of tumor markers related to DNA epigenetic modification, and the markers show a significant hypermethylation status in patients with tumor. The tumor markers can be used for clinical assisted screening, diagnosis, prognosis and the like of tumors, or can be used for designing diagnostic reagents and kits.

### BRIEF DESCRIPTION OF DRAWINGS

Figure 1 is a graph of average methylation values of SEQ ID NO.1 target sequence in TCGA database showed in Example 2 of the present disclosure;
Figure 2 is a graph of average methylation values of SEQ ID NO.3 target sequence in TCGA database showed in Example 2 of the present disclosure;
Figure 3 is a graph of average methylation values of SEQ ID NO.4 target sequence in TCGA database showed in Example 2 of the present disclosure;
Figure 4 is a graph of average methylation values of SEQ ID NO.5 target sequence in the TCGA database showed in Example 2 of the present disclosure;
Figure 5 is a graph of average methylation values of SEQ ID NO.6 target sequence in the TCGA database showed in Example 2 of the present disclosure;

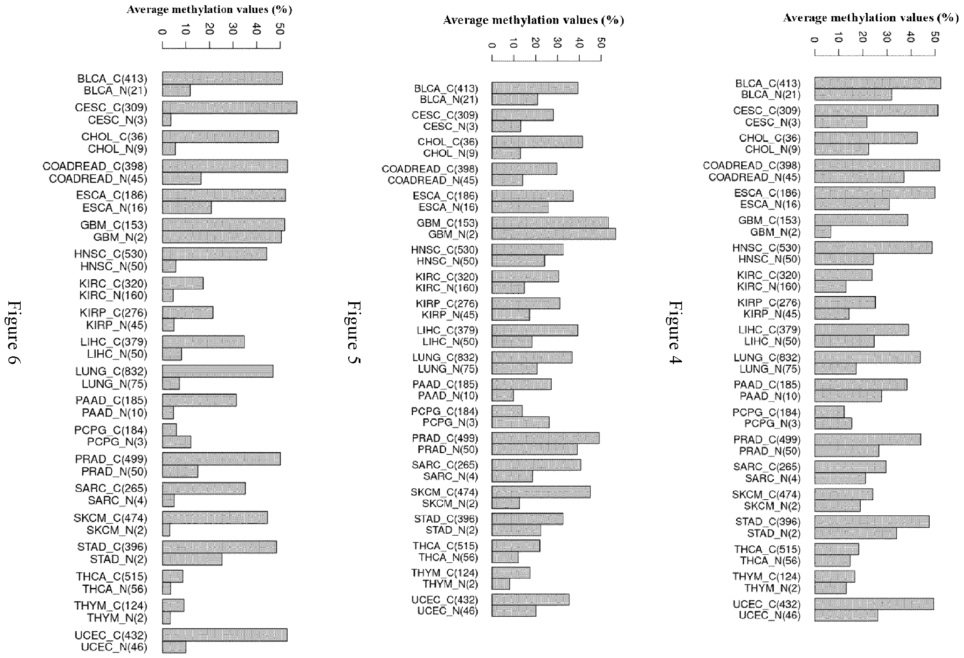

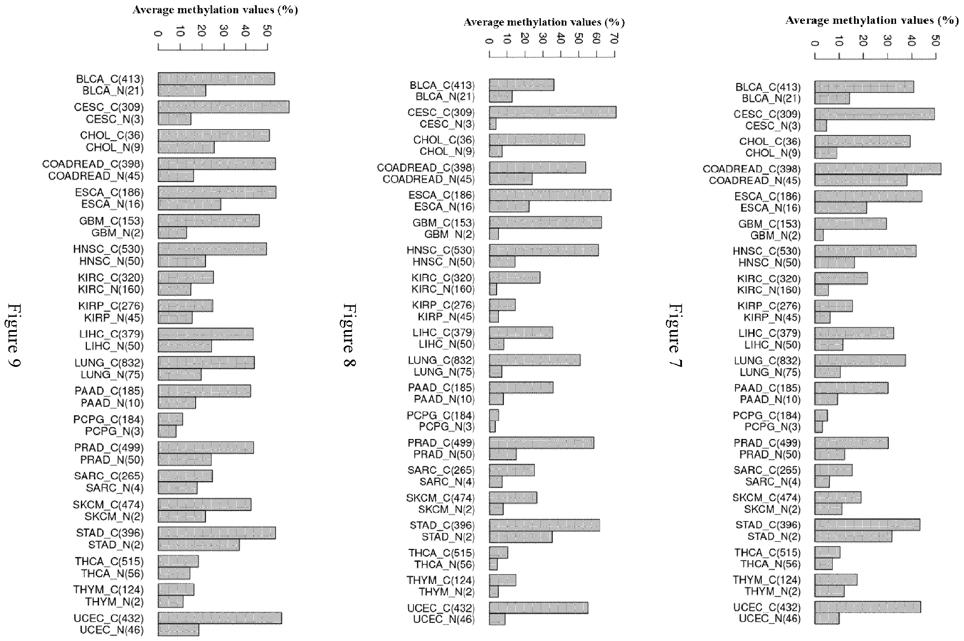

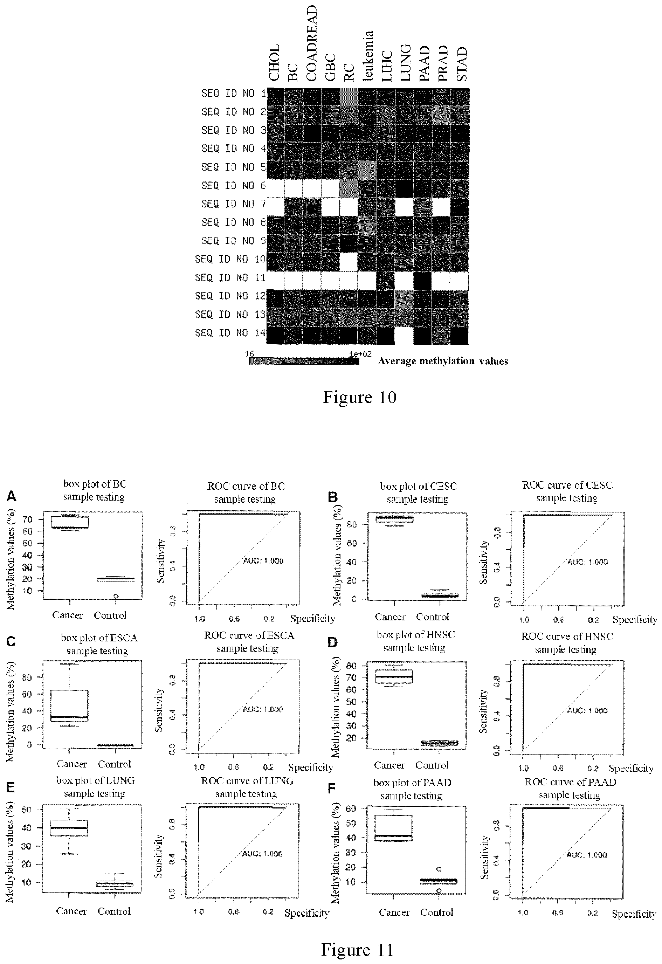

Figure 6 is a graph of average methylation values of SEQ ID NO.8 target sequence in TCGA database showed in Example 2 of the present disclosure;Figure 7 is a graph of average methylation values of SEQ ID NO. 10 target sequence in TCGA database showed in Example 2 of the present disclosure;Figure 8 is a graph of average methylation values of SEQ ID NO. 12 target sequence in TCGA database showed in Example 2 of the present disclosure;Figure 9 is a graph of average methylation values of SEQ ID NO. 13 target sequence in TCGA database showed in Example 2 of the present disclosure;Figure 10 is a heat map of average methylation values of SEQ ID NO.1-SEQ ID NO. 14 target sequences on different tumor cell lines in Example 3 of the present disclosure;
Figure 11 is a graph of average methylation value distribution and ROC of SEQ ID NO.1 target sequence on six tumors in Example 4 of the present disclosure;
Figure 12 is a graph of average methylation value distribution and ROC of SEQ ID NO.2 target sequence on six tumors in Example 5 of the present disclosure;
Figure 13 is a graph of average methylation value distribution and ROC of SEQ ID NO.3 target sequence on six tumors in Example 6 of the present disclosure;
Figure 14 is a graph of average methylation value distribution and ROC of SEQ ID NO.4 target sequence on six tumors in Example 7 of the present disclosure;
Figure 15 is a graph of average methylation value distribution and ROC of SEQ ID NO.5 target sequence on six tumors in Example 8 of the present disclosure;
Figure 16 is a graph of average methylation value distribution and ROC of SEQ ID NO.6 target sequence on six tumors in Example 9 of the present disclosure;
Figure 17 is a graph of average methylation value distribution and ROC of SEQ ID NO.7 target sequence on six tumors in Example 10 of the present disclosure;
Figure 18 is a graph of average methylation value distribution and ROC of SEQ ID NO.8 target sequence on six tumors in Example 11 of the present disclosure;
Figure 19 is a graph of average methylation value distribution and ROC of SEQ ID NO.9 target sequence on six tumors in Example 12 of the present disclosure;
Figure 20 is a graph of average methylation value distribution and ROC of SEQ ID NO.10 target sequence on six tumors in Example 13 of the present disclosure;
Figure 21 is a graph of average methylation value distribution and ROC of SEQ ID NO.11 target sequence on six tumors in Example 14 of the present disclosure;
Figure 22 is a graph of average methylation value distribution and ROC of SEQ ID NO. 12 target sequence on six tumors in Example 15 of the present disclosure;
Figure 23 is a graph of average methylation value distribution and ROC of SEQ ID NO.13 target sequence in six tumors in Example 16 of the present disclosure;
Figure 24 is a graph of average methylation value distribution and ROC of SEQ ID NO.14 target sequence on six tumors in Example 17 of the present disclosure.

### DETAILED DESCRIPTION

Detailed description of the present disclosure can be further illustrated by the specific examples and drawings described below. It should be understood that these examples are merely illustrative, and do not limit the scope of the present disclosure. The experimental methods without specifying the specific conditions in the following examples generally used the conventional methods and conditions or followed the manufacturer's recommendation.

### Example 1. Detection of methylation levels

RRBS (Reduced Representation Bisulfite Sequencing), WGBS (Whole Genome Bisulfite Sequencing) and other methylation detecting methods were used, mainly comprising the following steps:
1.1. Obtaining samples: Tumor cell lines were selected, or cancer tissues and adjacent normal tissues were obtained in clinical.
1.2. A DNA extraction kit was used to extract the DNA of samples.
1.3. Extracted DNA were performed a whole genome sequencing .
1.4. After sequencing, the sequences were aligned to the target sequences, and the methylation status of each CpG site in the target sequence region was calculated. Average methylation value of the sequenced CpG site in the target sequence was calculated and used as the average methylation value of the target sequence of the sample.

### Example 2. Verification of SEQ ID NO.1-SEQ ID NO.14 target sequences with methylation chip data in TCGA database.

### 2.1. Target sequences and databases

Target sequences of SEQ ID NO.1-SEQ ID NO. 14 are shown in Table 1.

By using TCGA database, the DNA methylation microarray data of more than 20 types of cancer tissues and their adjacent normal tissues were collected, comprising: bladder cancer (BLCA), cervical cancer (CESC), cholangiocarcinoma (CHOL), colon adenocarcinoma (COADREAD), esophageal cancer (ESCA), glioblastoma (GBM), head and neck squamous cell carcinoma (HNSC), kidney renal clear cell carcinoma (KIRC), kidney renal papillary cell carcinoma (KIRP), liver hepatocellular carcinoma (LIHC), Lung adenocarcinoma (LUNG), pancreatic adenocarcinoma (PAAD), pheochromocytoma and paraganglioma (PCPG), prostate adenocarcinoma (PRAD), sarcoma (SARC), skin cutaneous melanoma (SKCM), stomach adenocarcinoma (STAD), thyroid carcinoma (THCA), thymoma (THYM), and uterine corpus endometrial carcinoma (UCEC).

2.2. The number of probes contained in each target sequence was obtained and the results are shown in Table 3;

**Table 3 The number of probes contained in each target sequence**

| SEQ ID No | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| Probe Number | 1 | 0 | 1 | 2 | 1 | 1 | 0 | 1 | 0 | 1 | 0 | 1 | 2 | 0 |

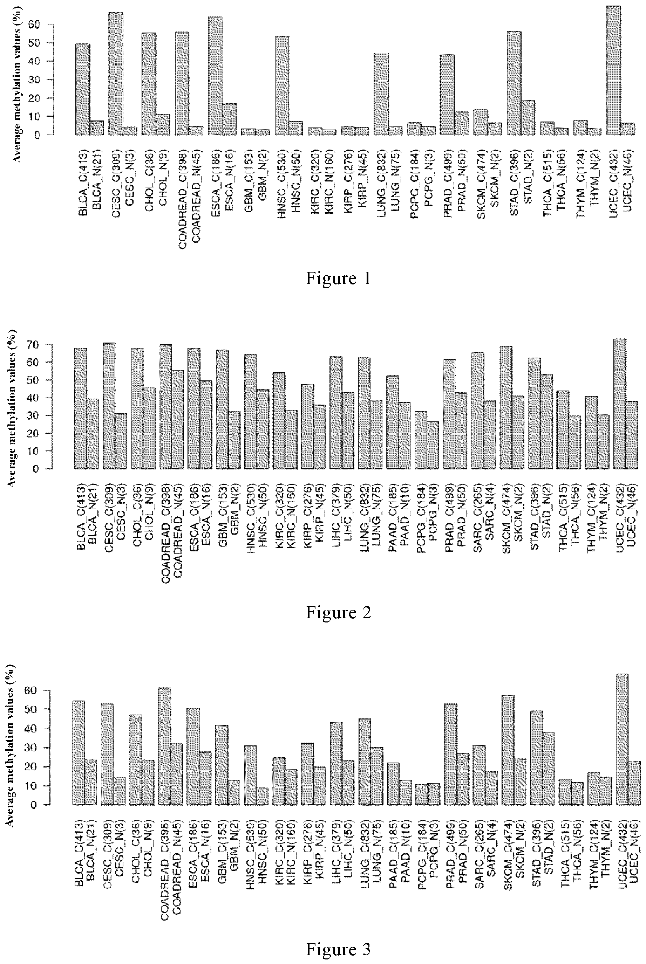

2.3. Average methylation values of all probes contained in target sequences in all cancer samples and normal tissue samples were calculated according to the method in Example 1. The results are shown in Figure 1-9. Naming method of samples is: English name of cancer is _C or N (number), wherein the C represents cancer, N represents normal tissue, and the number represents the sample size.

Figure 1 shows average methylation values of SEQ ID NO 1 target sequence in the TCGA database. It shows that average methylation values of SEQ ID NO 1 target sequence were obviously higher in 17 types of cancers including bladder cancer (BLCA), cervical cancer (CESC), cholangiocarcinoma (CHOL), colon adenocarcinoma (COADREAD), esophageal cancer (ESCA), glioblastoma (GBM), head and neck squamous cell carcinoma (HNSC), kidney renal clear cell carcinoma (KIRC), kidney renal papillary cell carcinoma (KIRP), lung adenocarcinoma (LUNG), pheochromocytoma and paraganglioma (PCPG), prostate adenocarcinoma (PRAD), skin cutaneous melanoma (SKCM), stomach adenocarcinoma (STAD), thyroid carcinoma (THCA), thymoma (THYM), and uterine corpus endometrial carcinoma (UCEC) than that in normal tissues.

Figure 2 shows average methylation values of SEQ ID NO 3 target sequence in the TCGA database. It shows that average methylation values of target sequences of SEQ ID NO 3 were higher in 20 types of cancers including bladder cancer (BLCA), cervical cancer (CESC), cholangiocarcinoma (CHOL), colon adenocarcinoma (COADREAD), esophageal cancer (ESCA), glioblastoma (GBM), head and neck squamous cell carcinoma (HNSC), kidney renal clear cell carcinoma (KIRC), kidney renal papillary cell carcinoma (KIRP), liver hepatocellular carcinoma (LIHC), lung adenocarcinoma (LUNG), pancreatic adenocarcinoma (PAAD), pheochromocytoma and paraganglioma (PCPG), prostate adenocarcinoma (PRAD), sarcoma (SARC), skin cutaneous melanoma (SKCM), stomach adenocarcinoma (STAD), thyroid carcinoma (THCA), thymoma (THYM), and uterine corpus endometrial carcinoma (UCEC) than that in normal tissues.

Figure 3 shows average methylation values of SEQ ID NO 4 target sequence in the TCGA database. It shows that average methylation values of target sequences of SEQ ID NO 4 were obviously higher in 19 types of cancers including bladder cancer (BLCA), cervical cancer (CESC), cholangiocarcinoma (CHOL), colon adenocarcinoma (COADREAD), esophageal cancer (ESCA), glioblastoma (GBM), head and neck squamous cell carcinoma (HNSC), kidney renal clear cell carcinoma (KIRC), kidney renal papillary cell carcinoma (KIRP), liver hepatocellular carcinoma (LIHC), lung adenocarcinoma (LUNG), pancreatic adenocarcinoma (PAAD), prostate adenocarcinoma (PRAD), sarcoma (SARC), skin cutaneous melanoma (SKCM), stomach adenocarcinoma (STAD), thyroid carcinoma (THCA), thymoma (THYM), and uterine corpus endometrial carcinoma (UCEC) than that in normal tissues. The average methylation values of target sequence of SEQ ID NO 4 in pheochromocytoma and paraganglioma (PCPG) was almost indistinguishable from that in normal tissues.

Figure 4 shows average methylation values of SEQ ID NO 5 target sequence in the TCGA database. It shows that average methylation values of target sequences of SEQ ID NO 5 were obviously higher in 19 types of cancers including bladder cancer (BLCA), cervical cancer (CESC), cholangiocarcinoma (CHOL), colon adenocarcinoma (COADREAD), esophageal cancer (ESCA), glioblastoma (GBM), head and neck squamous cell carcinoma (HNSC), kidney renal clear cell carcinoma (KIRC), kidney renal papillary cell carcinoma (KIRP), liver hepatocellular carcinoma (LIHC), lung adenocarcinoma (LUNG), pancreatic adenocarcinoma (PAAD), prostate adenocarcinoma (PRAD), sarcoma (SARC), skin cutaneous melanoma (SKCM), stomach adenocarcinoma (STAD), thyroid carcinoma (THCA), thymoma (THYM), and uterine corpus endometrial carcinoma (UCEC) than that in normal tissues. Average methylation values of target sequence of SEQ ID NO 5 in pheochromocytoma and paraganglioma (PCPG) were lower than that in normal tissues.

Figure 5 shows average methylation values of SEQ ID NO 6 target sequence in the TCGA database. It shows that the average methylation value of target sequences of SEQ ID NO 6 were obviously higher in 18 types of cancers including bladder cancer (BLCA), cervical cancer (CESC), cholangiocarcinoma (CHOL), colon adenocarcinoma (COADREAD), esophageal cancer (ESCA), head and neck squamous cell carcinoma (HNSC), kidney renal clear cell carcinoma (KIRC), kidney renal papillary cell carcinoma (KIRP), liver hepatocellular carcinoma (LIHC), lung adenocarcinoma (LUNG), pancreatic adenocarcinoma (PAAD), prostate adenocarcinoma (PRAD), sarcoma (SARC), skin cutaneous melanoma (SKCM), stomach adenocarcinoma (STAD), thyroid carcinoma (THCA), thymoma (THYM), and uterine corpus endometrial carcinoma (UCEC) than that in normal tissues. Average methylation values of SEQ ID NO 6 target sequence in glioblastoma (GBM), pheochromocytoma and paraganglioma (PCPG) were lower than that in normal tissues.

Figure 6 shows average methylation values of SEQ ID NO 8 target sequence in the TCGA database. It shows that average methylation values of SEQ ID NO 8 target sequence were obviously higher in 19 types of cancers including bladder cancer (BLCA), cervical cancer (CESC), cholangiocarcinoma (CHOL), colon adenocarcinoma (COADREAD), esophageal cancer (ESCA), glioblastoma (GBM), head and neck squamous cell carcinoma (HNSC), kidney renal clear cell carcinoma (KIRC), kidney renal papillary cell carcinoma (KIRP), liver hepatocellular carcinoma (LIHC), lung adenocarcinoma (LUNG), pancreatic adenocarcinoma (PAAD), prostate adenocarcinoma (PRAD), sarcoma (SARC), skin cutaneous melanoma (SKCM), stomach adenocarcinoma (STAD), thyroid carcinoma (THCA), thymoma (THYM), and uterine corpus endometrial carcinoma (UCEC) than that in normal tissues. Average methylation values of SEQ ID NO 8 target sequence in pheochromocytoma and paraganglioma (PCPG) were lower than that in normal tissues.

Figure 7 shows average methylation values of SEQ ID NO 10 target sequence in the TCGA database. It shows that average methylation values of SEQ ID NO 10 target sequence were higher in 20 types of cancers including bladder cancer (BLCA), cervical cancer (CESC), cholangiocarcinoma (CHOL), colon adenocarcinoma (COADREAD), esophageal cancer (ESCA), glioblastoma (GBM), head and neck squamous cell carcinoma (HNSC), kidney renal clear cell carcinoma (KIRC), kidney renal papillary cell carcinoma (KIRP), liver hepatocellular carcinoma (LIHC), lung adenocarcinoma (LUNG), pancreatic adenocarcinoma (PAAD), pheochromocytoma and paraganglioma (PCPG), prostate adenocarcinoma (PRAD), sarcoma (SARC), skin cutaneous melanoma (SKCM), stomach adenocarcinoma (STAD), thyroid carcinoma (THCA), thymoma (THYM), and uterine corpus endometrial carcinoma (UCEC) than that in normal tissues.

Figure 8 shows average methylation values of SEQ ID NO 12 target sequence in the TCGA database. It shows that average methylation values of SEQ ID NO 12 target sequence were higher in 20 types of cancers including bladder cancer (BLCA), cervical cancer (CESC), cholangiocarcinoma (CHOL), colon adenocarcinoma (COADREAD), esophageal cancer (ESCA), glioblastoma (GBM), head and neck squamous cell carcinoma (HNSC), kidney renal clear cell carcinoma (KIRC), kidney renal papillary cell carcinoma (KIRP), liver hepatocellular carcinoma (LIHC), lung adenocarcinoma (LUNG), pancreatic adenocarcinoma (PAAD), pheochromocytoma and paraganglioma (PCPG), prostate adenocarcinoma (PRAD), sarcoma (SARC), skin cutaneous melanoma (SKCM), stomach adenocarcinoma (STAD), thyroid carcinoma (THCA), thymoma (THYM), and uterine corpus endometrial carcinoma (UCEC) than that in normal tissues.

Figure 9 shows average methylation values of SEQ ID NO 13 target sequence in the TCGA database. It shows that average methylation values of SEQ ID NO 13 target sequence were higher in 20 types of cancers including bladder cancer (BLCA), cervical cancer (CESC), cholangiocarcinoma (CHOL), colon adenocarcinoma (COADREAD), esophageal cancer (ESCA), glioblastoma (GBM), head and neck squamous cell carcinoma (HNSC), kidney renal clear cell carcinoma (KIRC), kidney renal papillary cell carcinoma (KIRP), liver hepatocellular carcinoma (LIHC), lung adenocarcinoma (LUNG), pancreatic adenocarcinoma (PAAD), pheochromocytoma and paraganglioma (PCPG), prostate adenocarcinoma (PRAD), sarcoma (SARC), skin cutaneous melanoma (SKCM), stomach adenocarcinoma (STAD), thyroid carcinoma (THCA), thymoma (THYM), and uterine corpus endometrial carcinoma (UCEC) than that in normal tissues.

It shows that average methylation values of SEQ ID NO.1, SEQ ID NO.3, SEQ ID NO.4, SEQ ID NO.5, SEQ ID NO.6, SEQ ID NO.8, SEQ ID NO.10, SEQ ID NO.12, SEQ ID NO.13 target sequences in vast majority of the above tumors were higher than that in normal tissues.

### Example 3 Methylation levels of SEQ ID NO.1-SEQ ID NO.14 target sequences in tumor cell lines.

Eleven samples of tumor cell lines were collected, including cholangiocarcinoma, breast cancer, colon adenocarcinoma, gallbladder cancer (GBC), renal cancer (RC), leukemia, liver hepatocellular carcinoma, lung adenocarcinoma, pancreatic adenocarcinoma, prostate adenocarcinoma and stomach adenocarcinoma, respectively. According to the method of Example 1, the RRBS methylation sequencing was used to obtain average methylation values of 14 target sequences in each sample. The results are shown in Figure 10. White in the figure means that it was not detected or the sequencing quality was not up to standard. The darker the color, the higher the average methylation value. It shows that the methylation levels of SEQ ID NO.1, SEQ ID NO.2, SEQ ID NO.3, SEQ ID NO.4, SEQ ID NO.5, SEQ ID NO.8, SEQ ID NO.9, SEQ ID NO. 12 and SEQ ID NO. 13 target sequences could be detected in all 11 tumor cell lines, and average methylation values in the 11 tumor cell lines were high. The methylation levels of SEQ ID NO.6, SEQ ID NO.7, SEQ ID NO. 10, SEQ ID NO.11 and SEQ ID NO. 14 target sequences were detected only in some tumor cell lines.

### Example 4 Application of SEQ ID NO.1 target sequence in clinical detection

Samples: Samples were from 6 kinds of tumor tissues and their adjacent normal tissues, including breast cancer (5 cases of cancer tissues, 5 cases of adjacent normal tissues), cervical cancer (4 cases of cancer tissues, 5 cases of adjacent normal tissues), esophageal cancer (3 cases of cancer tissues, 1 case of adjacent normal tissues), head and neck cancer (4 cases of cancer tissues, 5 cases of adjacent normal tissues), lung adenocarcinoma (5 cases of cancer tissues, 5 cases of adjacent normal tissues) and pancreatic adenocarcinoma (5 cases of cancer tissues, 5 cases of adjacent normal tissues), respectively.

According to the method of Example 1, the RRBS methylation sequencing was used to obtain average methylation values of SEQ ID NO.1 target sequence in these 26 tumor tissues and 26 adjacent normal tissues, respectively. The results are shown in Figure 11. The figure comprises A, B, C, D, E, F, 6 groups of figures, in total. Each group of figures comprises 2 figures, wherein the left figure represents a box plot of average methylation values of target sequences in tumor samples and normal samples (control), showing the difference of methylation between tumor samples and normal samples; the right figure represents a ROC curve of the test, showing the specificity of differences. Wherein, A: samples of breast cancer and the control thereof, B: samples of cervical cancer and the control thereof, C: samples of esophageal cancer and the control thereof, D: samples of head and neck cancer and the control thereof, E: samples of lung adenocarcinoma and the control thereof, F: samples of pancreatic adenocarcinoma and the control thereof. It shows that in the 6 types of tumors, average methylation values of SEQ ID NO.1 target sequence in tumor samples was higher than that in adjacent normal tissues. According to average methylation values of SEQ ID NO.1 target sequence in samples, it can be accurately distinguished tumor samples from normal tissues.

### Example 5 Application of SEQ ID NO.2 target sequence in clinical detection

Samples: Samples were from 6 kinds of tumor tissues and their adjacent normal tissues, including breast cancer (4 cases of cancer tissues, 5 cases of adjacent normal tissues), cervical cancer (4 cases of cancer tissues, 5 cases of adjacent normal tissues), esophageal cancer (3 cases of cancer tissues, 1 case of adjacent normal tissues), head and neck cancer (4 cases of cancer tissues, 5 cases of adjacent normal tissues), lung adenocarcinoma (5 cases of cancer tissues, 5 cases of adjacent normal tissues) and pancreatic adenocarcinoma (5 cases of cancer tissues, 5 cases of adjacent normal tissues), respectively.

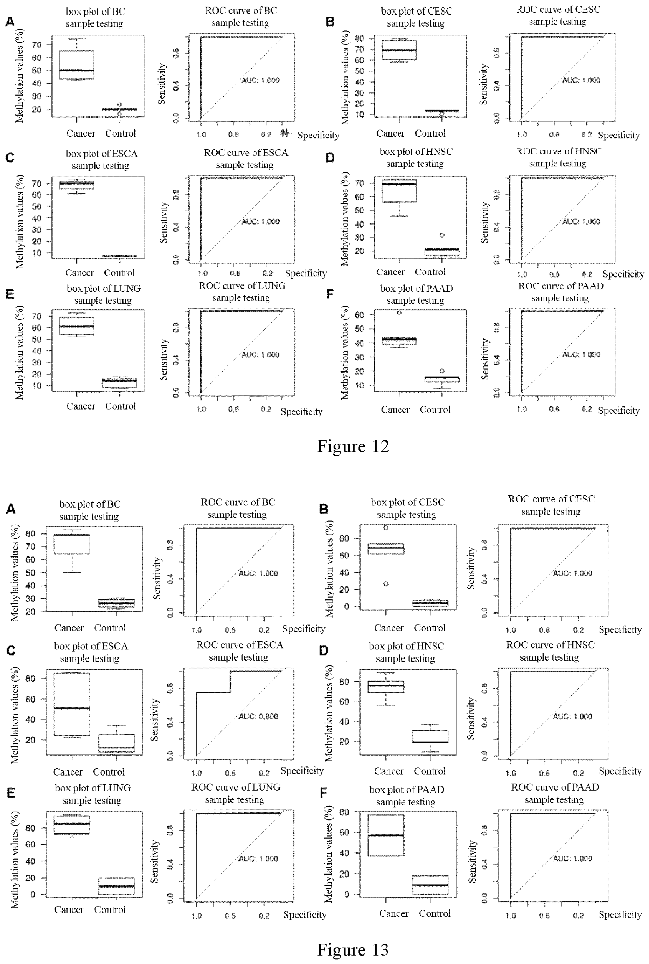

According to the method of Example 1, the RRBS methylation sequencing was used to obtain average methylation values of SEQ ID NO.2 target sequence in these 25 tumor tissues and 26 adjacent normal tissues, respectively. The results are shown in Figure 12. The figure comprises A, B, C, D, E, F, 6 groups of pictures, in total. Each group of figures comprises 2 figures, wherein the left figure represents a box plot of average methylation values of target sequences in tumor samples and normal samples (control), showing the difference of methylation between tumor samples and normal samples; the right figure represents a ROC curve of the test, showing the specificity of differences. Wherein, A: samples of breast cancer and the control thereof, B: samples of cervical cancer and the control thereof, C: samples of esophageal cancer and the control thereof, D: samples of head and neck cancer and the control thereof, E: samples of lung adenocarcinoma and the control thereof, F: samples of pancreatic adenocarcinoma and the control thereof. It shows that in the 6 types of tumors, average methylation values of SEQ ID NO.2 target sequence in tumor samples was higher than that in adjacent normal tissues. According to average methylation values of SEQ ID NO.2 target sequence in samples, it can be accurately distinguished tumor samples from normal tissues.

### Example 6 Application of SEQ ID NO.3 target sequence in clinical detection

Samples: Samples were from 6 kinds of tumor tissues and their adjacent normal tissues, including breast cancer (5 cases of cancer tissues, 5 cases of adjacent normal tissues), cervical cancer (5 cases of cancer tissues, 5 cases of adjacent normal tissues), esophageal cancer (5 cases of cancer tissues, 4 cases of adjacent normal tissues), head and neck cancer (5 cases of cancer tissues, 5 cases of adjacent normal tissues), lung adenocarcinoma (4 cases of cancer tissues, 2 cases of adjacent normal tissues) and pancreatic adenocarcinoma (2 cases of cancer tissues, 2 cases of adjacent normal tissues), respectively.

According to the method of Example 1, the RRBS methylation sequencing was used to obtain average methylation values of SEQ ID NO.3 target sequence in these 26 tumor tissues and 23 adjacent normal tissues, respectively. The results were shown in Figure 13. The figure comprises A, B, C, D, E, F, 6 groups of pictures, in total. Each group of figures comprises 2 figures, wherein the left figure represents a box plot of average methylation values of target sequences in tumor samples and normal samples (control), showing the difference of methylation between tumor samples and normal samples; the right figure represents a ROC curve of the test, showing the specificity of differences. Wherein, A: samples of breast cancer and the control thereof, B: samples of cervical cancer and the control thereof, C: samples of esophageal cancer and the control thereof, D: samples of head and neck cancer and the control thereof, E: samples of lung adenocarcinoma and the control thereof, F: samples of pancreatic adenocarcinoma and the control thereof. It shows that in the 5 types of tumors, average methylation values of SEQ ID NO.3 target sequence in tumor samples were higher than that in adjacent normal tissues; In esophageal cancer, average methylation values of SEQ ID NO.3 target sequence in most tumor samples were higher than that in adjacent normal tissues. According to average methylation values of SEQ ID NO.3 target sequence in samples, it can be accurately distinguished in vast majority of the tumor samples from normal tissues.

### Example 7 Application of SEQ ID NO.4 target sequence in clinical detection

Samples: Samples were from 6 kinds of tumor tissues and their adjacent normal tissues, including breast cancer (4 cases of cancer tissues, 4 cases of adjacent normal tissues), cervical cancer (5 cases of cancer tissues, 2 cases of adjacent normal tissues), esophageal cancer (5 cases of cancer tissues, 4 cases of adjacent normal tissues), head and neck cancer (2 cases of cancer tissues, 4 cases of adjacent normal tissues), lung adenocarcinoma (5 cases of cancer tissues, 5 cases of adjacent normal tissues) and pancreatic adenocarcinoma (5 cases of cancer tissues, 5 cases of adjacent normal tissues), respectively.

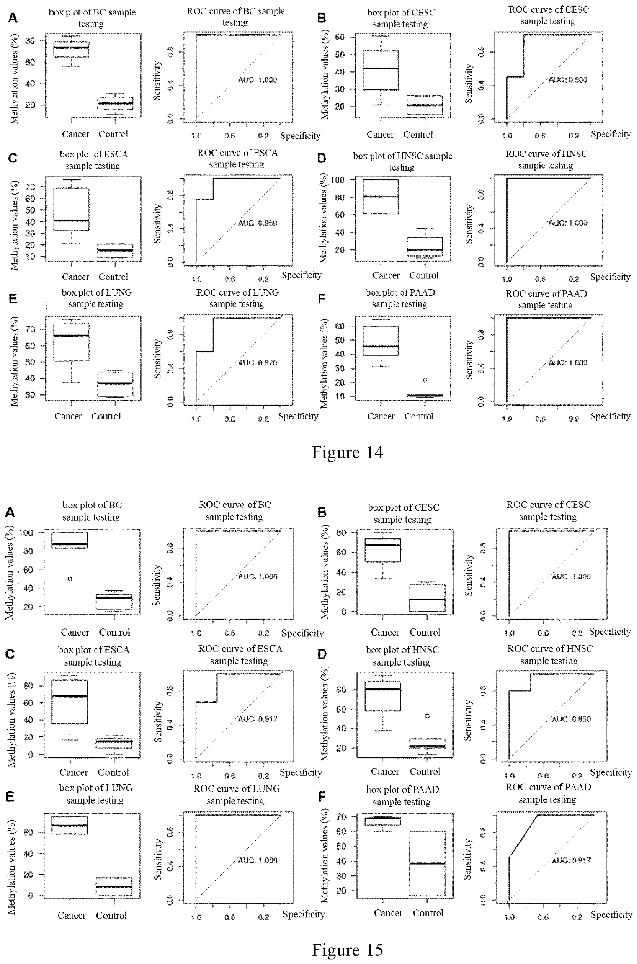

According to the method of Example 1, the RRBS methylation sequencing was used to obtain average methylation values of SEQ ID NO.4 target sequence in these 26 tumor tissues and 24 adjacent normal tissues, respectively. The results were shown in Figure 14. The figure comprises A, B, C, D, E, F, 6 groups of pictures, in total. Each group of figures comprises 2 figures, wherein the left figure represents a box plot of average methylation values of target sequences in tumor samples and normal samples (control), showing the difference of methylation between tumor samples and normal samples; the right figure represents a ROC curve of the test, showing the specificity of differences. Wherein, A: samples of breast cancer and the control thereof, B: samples of cervical cancer and the control thereof, C: samples of esophageal cancer and the control thereof, D: samples of head and neck cancer and the control thereof, E: samples of lung adenocarcinoma and the control thereof, F: samples of pancreatic adenocarcinoma and the control thereof. It shows that average methylation values of SEQ ID NO.4 target sequence in tumor samples of breast cancer, head and neck cancer and esophageal cancer were all higher than that in adjacent normal tissues; In tumor samples of esophageal cancer, cervical cancer and lung adenocarcinoma, average methylation value of SEQ ID NO.4 target sequence was mostly higher than that in adjacent normal tissues, with the mean also higher than that in adjacent normal tissues. According to average methylation values of SEQ ID NO.4 target sequence in samples, it can be accurately distinguished in vast majority of the tumor samples from normal tissues.

### Example 8 Application of SEQ ID NO.5 target sequence in clinical detection

Samples: Samples were from 6 kinds of tumor tissues and their adjacent normal tissues, including breast cancer (5 cases of cancer tissues, 5 cases of adjacent normal tissues), cervical cancer (3 cases of cancer tissues, 4 cases of adjacent normal tissues), esophageal cancer (4 cases of cancer tissues, 3 cases of adjacent normal tissues), head and neck cancer (4 cases of cancer tissues, 5 cases of adjacent normal tissues), lung adenocarcinoma (2 cases of cancer tissues, 2 cases of adjacent normal tissues) and pancreatic adenocarcinoma (3 cases of cancer tissues, 2 cases of adjacent normal tissues), respectively.

According to the method of Example 1, the RRBS methylation sequencing was used to obtain average methylation values of SEQ ID NO.5 target sequence in these 21 tumor tissues and 21 adjacent normal tissues, respectively. The results were shown in Figure 15. The figure comprises A, B, C, D, E, F, 6 groups of pictures, in total. Each group of figures comprises 2 figures, wherein the left figure represents a box plot of average methylation values of target sequences in tumor samples and normal samples (control), showing the difference of methylation between tumor samples and normal samples; the right figure represents a ROC curve of the test, showing the specificity of differences. Wherein, A: samples of breast cancer and the control thereof, B: samples of cervical cancer and the control thereof, C: samples of esophageal cancer and the control thereof, D: samples of head and neck cancer and the control thereof, E: samples of lung adenocarcinoma and the control thereof, F: samples of pancreatic adenocarcinoma and the control thereof. It shows that average methylation values of SEQ ID NO.5 target sequence in tumor samples of breast cancer, cervical cancer and lung adenocarcinoma were all higher than that in adjacent normal tissues. In tumor samples of esophageal cancer, head and neck cancer and lung adenocarcinoma, average methylation value of SEQ ID NO.5 target sequence was mostly higher than that in adjacent normal tissues, with the mean also higher than that in adjacent normal tissues. According to average methylation values of SEQ ID NO.5 target sequence in samples, it can be accurately distinguished in vast majority of the tumor samples from normal tissues.

### Example 9 Application of SEQ ID NO.6 target sequence in clinical detection

Samples: Samples were from 5 kinds of tumor tissues and their adjacent normal tissues, including breast cancer (5 cases of cancer tissues, 5 cases of adjacent normal tissues), esophageal cancer (2 cases of cancer tissues, 1 case of adjacent normal tissues), head and neck cancer (5 cases of cancer tissues, 5 cases of adjacent normal tissues), lung adenocarcinoma (3 cases of cancer tissues, 1 case of adjacent normal tissues) and pancreatic adenocarcinoma (2 cases of cancer tissues, 1 case of adjacent normal tissues), respectively.

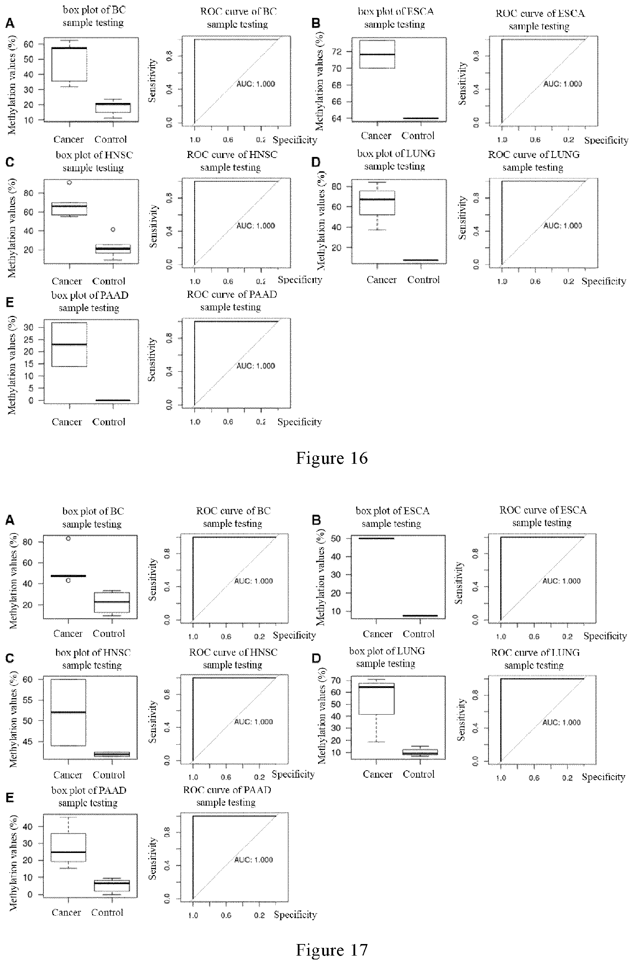

According to the method of Example 1, the RRBS methylation sequencing was used to obtain average methylation values of SEQ ID NO.6 target sequence in these 17 tumor tissues and 13 adjacent normal tissues, respectively. The results were shown in Figure 16. The figure comprises A, B, C, D, E, 5 groups of pictures, in total. Each group of figures comprises 2 figures, wherein the left figure represents a box plot of average methylation values of target sequences in tumor samples and normal samples (control), showing the difference of methylation between tumor samples and normal samples; the right figure represents a ROC curve of the test, showing the specificity of differences. Wherein, A: samples of breast cancer and the control thereof, B: samples of esophageal cancer and the control thereof, C: samples of head and neck cancer and the control thereof, D: samples of lung adenocarcinoma and the control thereof, E: samples of pancreatic adenocarcinoma and the control thereof. It shows that in the 5 types of tumors, average methylation values of SEQ ID NO.6 target sequence in tumor samples were higher than that in adjacent normal tissues. According to average methylation values of SEQ ID NO.6 target sequence in samples, it can be accurately distinguished tumor samples from normal tissues.

### Example 10 Application of SEQ ID NO.7 target sequence in clinical detection

Samples: Samples were from 5 kinds of tumor tissues and their adjacent normal tissues, including breast cancer (5 cases of cancer tissues, 4 cases of adjacent normal tissues), cervical cancer (2 cases of cancer tissues, 1 case of adjacent normal tissues), head and neck cancer (2 cases of cancer tissues, 2 cases of adjacent normal tissues), lung adenocarcinoma (3 cases of cancer tissues, 3 case of adjacent normal tissues) and pancreatic adenocarcinoma (4 cases of cancer tissues, 5 cases of adjacent normal tissues), respectively.

According to the method of Example 1, the RRBS methylation sequencing was used to obtain average methylation values of SEQ ID NO.7 target sequence in these 16 tumor tissues and 15 adjacent normal tissues, respectively. The results were shown in Figure 17. The figure comprises A, B, C, D, E, 5 groups of pictures, in total. Each group of figures comprises 2 figures, wherein the left figure represents a box plot of average methylation values of target sequences in tumor samples and normal samples (control), showing the difference of methylation between tumor samples and normal samples; the right figure represents a ROC curve of the test, showing the specificity of differences. Wherein, A: samples of breast cancer and the control thereof, B: samples of cervical cancer and the control thereof, C: samples of head and neck cancer and the control thereof, D: samples of lung adenocarcinoma and the control thereof, E: samples of pancreatic adenocarcinoma and the control thereof. It shows that in the 5 types of tumors, average methylation values of SEQ ID NO.7 target sequence in tumor samples were higher than that in adjacent normal tissues. According to average methylation values of SEQ ID NO.7 target sequence in samples, it can be accurately distinguished tumor samples from normal tissues.

### Example 11 Application of SEQ ID NO.8 target sequence in clinical detection

Samples: Samples were from 6 kinds of tumor tissues and their adjacent normal tissues, including breast cancer (5 cases of cancer tissues, 5 cases of adjacent normal tissues), cervical cancer (4 cases of cancer tissues, 5 cases of adjacent normal tissues), esophageal cancer (4 cases of cancer tissues, 5 cases of adjacent normal tissues), head and neck cancer (4 cases of cancer tissues, 5 cases of adjacent normal tissues), lung adenocarcinoma (5 cases of cancer tissues, 5 cases of adjacent normal tissues) and pancreatic adenocarcinoma (4 cases of cancer tissues, 5 cases of adjacent normal tissues), respectively.

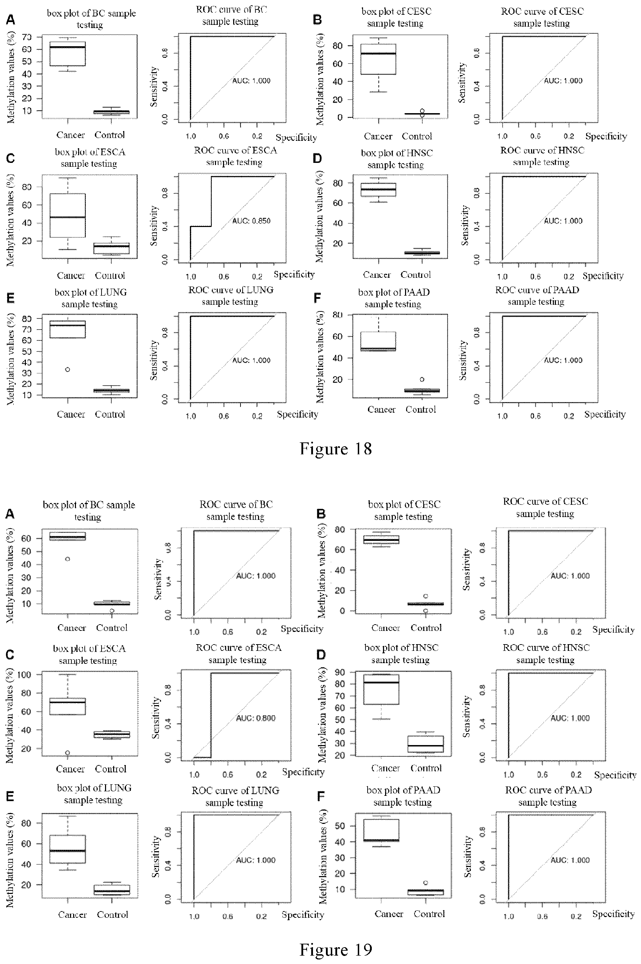

According to the method of Example 1, the RRBS methylation sequencing was used to obtain average methylation values of SEQ ID NO.8 target sequence in these 26 tumor tissues and 30 adjacent normal tissues, respectively. The results were shown in Figure 18. The figure comprises A, B, C, D, E, F, 6 groups of pictures, in total. Each group of figures comprises 2 figures, wherein the left figure represents a box plot of average methylation values of target sequences in tumor samples and normal samples (control), showing the difference of methylation between tumor samples and normal samples; the right figure represents a ROC curve of the test, showing the specificity of differences. Wherein, A: samples of breast cancer and the control thereof, B: samples of cervical cancer and the control thereof, C: samples of esophageal cancer and the control thereof, D: samples of head and neck cancer and the control thereof, E: samples of lung adenocarcinoma and the control thereof, F: samples of pancreatic adenocarcinoma and the control thereof. It shows that average methylation values of SEQ ID NO.8 target sequence in tumor samples of breast cancer, cervical cancer, lung adenocarcinoma, head and neck cancer and pancreatic adenocarcinoma were all higher than that in adjacent normal tissues. In tumor samples of esophageal cancer, average methylation value of SEQ ID NO.8 target sequence was mostly higher than that in adjacent normal tissues, with the mean also higher than that in adjacent normal tissues. According to average methylation values of SEQ ID NO.8 target sequence in samples, it can be accurately distinguished in vast majority of the tumor samples from normal tissues.

### Example 12 Application of SEQ ID NO.9 target sequence in clinical detection

Samples: Samples were from 6 kinds of tumor tissues and their adjacent normal tissues, including breast cancer (5 cases of cancer tissues, 5 cases of adjacent normal tissues), cervical cancer (4 cases of cancer tissues, 5 cases of adjacent normal tissues), esophageal cancer (5 cases of cancer tissues, 4 cases of adjacent normal tissues), head and neck cancer (4 cases of cancer tissues, 4 cases of adjacent normal tissues), lung adenocarcinoma (5 cases of cancer tissues, 4 cases of adjacent normal tissues) and pancreatic adenocarcinoma (5 cases of cancer tissues, 5 cases of adjacent normal tissues), respectively.

According to the method of Example 1, the RRBS methylation sequencing was used to obtain average methylation values of SEQ ID NO.9 target sequence in these 28 tumor tissues and 27 adjacent normal tissues, respectively. The results were shown in Figure 19. The figure comprises A, B, C, D, E, F, 6 groups of pictures, in total. Each group of figures comprises 2 figures, wherein the left figure represents a box plot of average methylation values of target sequences in tumor samples and normal samples (control), showing the difference of methylation between tumor samples and normal samples; the right figure represents a ROC curve of the test, showing the specificity of differences. Wherein, A: samples of breast cancer and the control thereof, B: samples of cervical cancer and the control thereof, C: samples of esophageal cancer and the control thereof, D: samples of head and neck cancer and the control thereof, E: samples of lung adenocarcinoma and the control thereof, F: samples of pancreatic adenocarcinoma and the control thereof. It shows that average methylation values of SEQ ID NO.9 target sequence in tumor samples of breast cancer, cervical cancer, lung adenocarcinoma, head and neck cancer and pancreatic adenocarcinoma were all higher than that in adjacent normal tissues. In tumor samples of esophageal cancer, average methylation value of SEQ ID NO.9 target sequence was mostly higher than that in adjacent normal tissues, with the mean also higher than that in adjacent normal tissues. According to average methylation values of SEQ ID NO.9 target sequence in samples, it can be accurately distinguished in vast majority of the tumor samples from normal tissues.

### Example 13 Application of SEQ ID NO.10 target sequence in clinical detection

Samples: Samples were from 6 kinds of tumor tissues and their adjacent normal tissues, including breast cancer (5 cases of cancer tissues, 5 cases of adjacent normal tissues), cervical cancer (4 cases of cancer tissues, 3 cases of adjacent normal tissues), esophageal cancer (4 cases of cancer tissues, 5 cases of adjacent normal tissues), head and neck cancer (4 cases of cancer tissues, 5 cases of adjacent normal tissues), lung adenocarcinoma (4 cases of cancer tissues, 3 cases of adjacent normal tissues) and pancreatic adenocarcinoma (4 cases of cancer tissues, 5 cases of adjacent normal tissues), respectively.

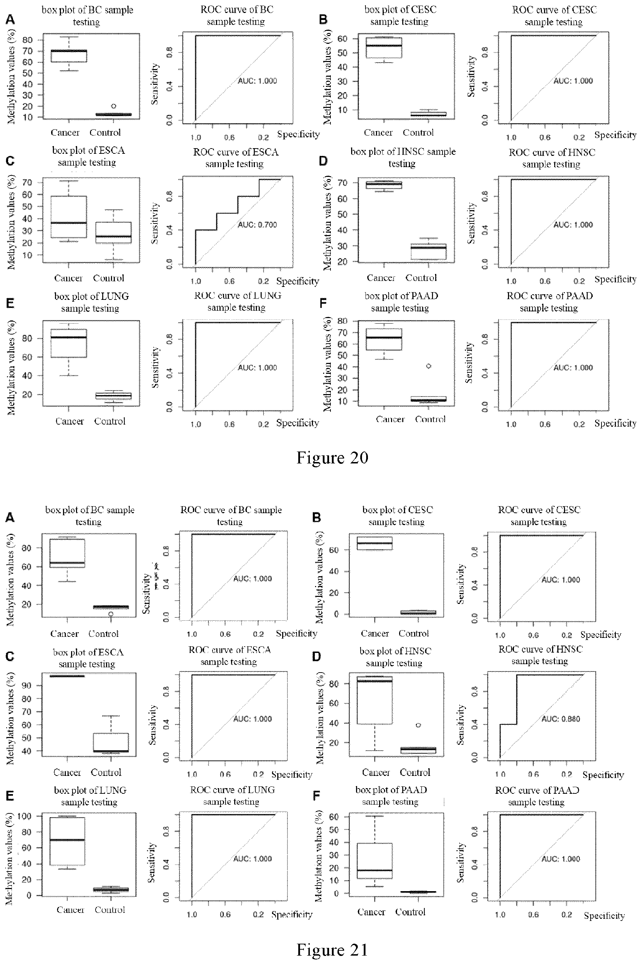

According to the method of Example 1, the RRBS methylation sequencing was used to obtain average methylation values of SEQ ID NO. 10 target sequence in these 25 tumor tissues and 26 adjacent normal tissues, respectively. The results were shown in Figure 20. The figure comprises A, B, C, D, E, F, 6 groups of pictures, in total. Each group of figures comprises 2 figures, wherein the left figure represents a box plot of average methylation values of target sequences in tumor samples and normal samples (control), showing the difference of methylation between tumor samples and normal samples; the right figure represents a ROC curve of the test, showing the specificity of differences. Wherein, A: samples of breast cancer and the control thereof, B: samples of cervical cancer and the control thereof, C: samples of esophageal cancer and the control thereof, D: samples of head and neck cancer and the control thereof, E: samples of lung adenocarcinoma and the control thereof, F: samples of pancreatic adenocarcinoma and the control thereof. It shows that average methylation values of SEQ ID NO.10 target sequence in tumor samples of breast cancer, cervical cancer, lung adenocarcinoma, head and neck cancer and pancreatic adenocarcinoma were all higher than that in adjacent normal tissues. In tumor samples of esophageal cancer, average methylation value of SEQ ID NO.10 target sequence was mostly higher than that in adjacent normal tissues, with the mean also higher than that in adjacent normal tissues. According to average methylation values of SEQ ID NO.10 target sequence in samples, it can be accurately distinguished in vast majority of the tumor samples from adjacent normal tissues.

### Example 14 Application of SEQ ID NO.11 target sequence in clinical detection

Samples: Samples were from 6 kinds of tumor tissues and their adjacent normal tissues, including breast cancer (5 cases of cancer tissues, 5 cases of adjacent normal tissues), cervical cancer (2 cases of cancer tissues, 5 cases of adjacent normal tissues), esophageal cancer (1 case of cancer tissues, 3 cases of adjacent normal tissues), head and neck cancer (5 cases of cancer tissues, 5 cases of adjacent normal tissues), lung adenocarcinoma (4 cases of cancer tissues, 4 cases of adjacent normal tissues) and pancreatic adenocarcinoma (3 cases of cancer tissues, 3 cases of adjacent normal tissues), respectively.

According to the method of Example 1, the RRBS methylation sequencing was used to obtain average methylation values of SEQ ID NO.11 target sequence in these 20 tumor tissues and 25 adjacent normal tissues, respectively. The results were shown in Figure 21. The figure comprises A, B, C, D, E, F, 6 groups of pictures, in total. Each group of figures comprises 2 figures, wherein the left figure represents a box plot of average methylation values of target sequences in tumor samples and normal samples (control), showing the difference of methylation between tumor samples and normal samples; the right figure represents a ROC curve of the test, showing the specificity of differences. Wherein, A: samples of breast cancer and the control thereof, B: samples of cervical cancer and the control thereof, C: samples of esophageal cancer and the control thereof, D: samples of head and neck cancer and the control thereof, E: samples of lung adenocarcinoma and the control thereof, F: samples of pancreatic adenocarcinoma and the control thereof. It shows that average methylation values of SEQ ID NO.11 target sequence in tumor samples of breast cancer, cervical cancer, lung adenocarcinoma, esophageal cancer and pancreatic adenocarcinoma were all higher than that in adjacent normal tissues. In tumor samples of head and neck cancer, average methylation value of SEQ ID NO.11 target sequence was mostly higher than that in adjacent normal tissues, with the mean also higher than that in adjacent normal tissues. According to average methylation values of SEQ ID NO.11 target sequence in samples, it can be accurately distinguished in vast majority of the tumor samples from adjacent normal tissues.

### Example 15 Application of SEQ ID NO.12 target sequence in clinical detection

Samples: Samples were from 6 kinds of tumor tissues and their adjacent normal tissues, including breast cancer (5 cases of cancer tissues, 5 cases of adjacent normal tissues), cervical cancer (3 cases of cancer tissues, 5 cases of adjacent normal tissues), esophageal cancer (4 cases of cancer tissues, 3 cases of adjacent normal tissues), head and neck cancer (5 cases of cancer tissues, 5 cases of adjacent normal tissues), lung adenocarcinoma (5 cases of cancer tissues, 5 cases of adjacent normal tissues) and pancreatic adenocarcinoma (5 cases of cancer tissues, 5 cases of adjacent normal tissues), respectively.

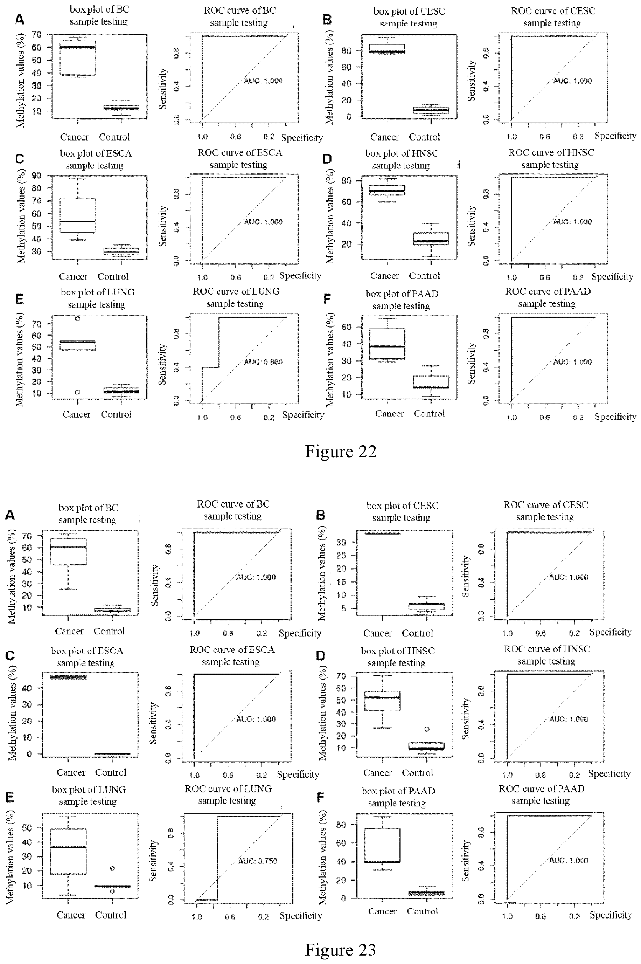

According to the method of Example 1, the RRBS methylation sequencing was used to obtain average methylation values of SEQ ID NO. 12 target sequence in these 27 tumor tissues and 28 adjacent normal tissues, respectively. The results were shown in Figure 22. The figure comprises A, B, C, D, E, F, 6 groups of pictures, in total. Each group of figures comprises 2 figures, wherein the left figure represents a box plot of average methylation values of target sequences in tumor samples and normal samples (control), showing the difference of methylation between tumor samples and normal samples; the right figure represents a ROC curve of the test, showing the specificity of differences. Wherein, A: samples of breast cancer and the control thereof, B: samples of cervical cancer and the control thereof, C: samples of esophageal cancer and the control thereof, D: samples of head and neck cancer and the control thereof, E: samples of lung adenocarcinoma and the control thereof, F: samples of pancreatic adenocarcinoma and the control thereof. It shows that average methylation values of SEQ ID NO. 12 target sequence in tumor samples of breast cancer, cervical cancer, head and neck cancer, esophageal cancer and pancreatic adenocarcinoma were all higher than that in adjacent normal tissues. In tumor samples of lung adenocarcinoma, average methylation value of SEQ ID NO. 12 target sequence was mostly higher than that in adjacent normal tissues, with the mean also higher than that in adjacent normal tissues. According to average methylation values of SEQ ID NO. 12 target sequence in samples, it can be accurately distinguished in vast majority of the tumor samples from normal tissues.

### Example 16 Application of SEQ ID NO.13 target sequence in clinical detection

Samples: Samples were from 6 kinds of tumor tissues and their adjacent normal tissues, including breast cancer (5 cases of cancer tissues, 5 cases of adjacent normal tissues), cervical cancer (1 case of cancer tissues, 5 cases of adjacent normal tissues), esophageal cancer (2 cases of cancer tissues, 1 case of adjacent normal tissues), head and neck cancer (5 cases of cancer tissues, 5 cases of adjacent normal tissues), lung adenocarcinoma (4 cases of cancer tissues, 5 cases of adjacent normal tissues) and pancreatic adenocarcinoma (5 cases of cancer tissues, 5 cases of adjacent normal tissues), respectively.

According to the method of Example 1, the RRBS methylation sequencing was used to obtain average methylation values of SEQ ID NO. 13 target sequence in these 22 tumor tissues and 26 adjacent normal tissues, respectively. The results were shown in Figure 23. The figure comprises A, B, C, D, E, F, 6 groups of pictures, in total. Each group of figures comprises 2 figures, wherein the left figure represents a box plot of average methylation values of target sequences in tumor samples and normal samples (control), showing the difference of methylation between tumor samples and normal samples; the right figure represents a ROC curve of the test, showing the specificity of differences. Wherein, A: samples of breast cancer and the control thereof, B: samples of cervical cancer and the control thereof, C: samples of esophageal cancer and the control thereof, D: samples of head and neck cancer and the control thereof, E: samples of lung adenocarcinoma and the control thereof, F: samples of pancreatic adenocarcinoma and the control thereof. It shows that average methylation values of SEQ ID NO. 13 target sequence in tumor samples of breast cancer, cervical cancer, head and neck cancer, esophageal cancer and pancreatic adenocarcinoma were all higher than that in adjacent normal tissues. In tumor samples of lung adenocarcinoma, average methylation value of SEQ ID NO. 13 target sequence was mostly higher than that in adjacent normal tissues, with the mean also higher than that in adjacent normal tissues. According to average methylation values of SEQ ID NO. 13 target sequence in samples, it can be accurately distinguished in vast majority of the tumor samples from adjacent normal tissues.

### Example 17 Application of SEQ ID NO.14 target sequence in clinical detection

Samples: Samples were from 6 kinds of tumor tissues and their adjacent normal tissues, including breast cancer (5 cases of cancer tissues, 5 cases of adjacent normal tissues), cervical cancer (3 cases of cancer tissues, 5 cases of adjacent normal tissues), esophageal cancer (3 cases of cancer tissues, 1 case of adjacent normal tissues), head and neck cancer (4 cases of cancer tissues, 5 cases of adjacent normal tissues), lung adenocarcinoma (3 cases of cancer tissues, 3 cases of adjacent normal tissues) and pancreatic adenocarcinoma (2 cases of cancer tissues, 2 cases of adjacent normal tissues), respectively.

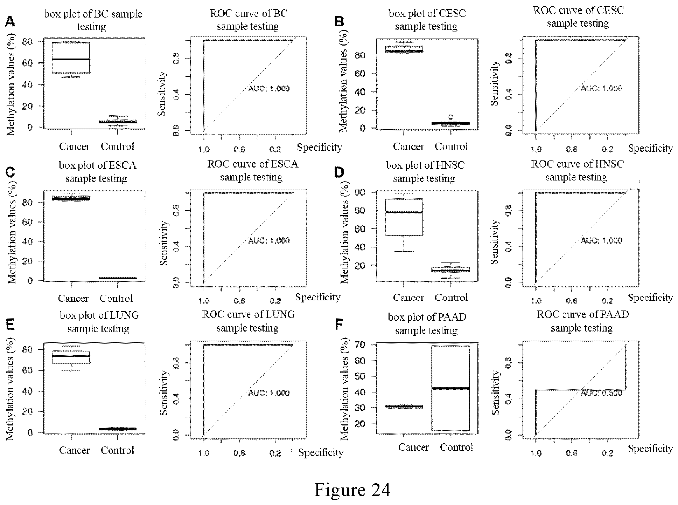

According to the method of Example 1, the RRBS methylation sequencing was used to obtain average methylation values of SEQ ID NO. 14 target sequence in these 20 tumor tissues and 21 adjacent normal tissues, respectively. The results were shown in Figure 24. The figure comprises A, B, C, D, E, F, 6 groups of pictures, in total. Each group of figures comprises 2 figures, wherein the left figure represents a box plot of average methylation values of target sequences in tumor samples and normal samples (control), showing the difference of methylation between tumor samples and normal samples; the right figure represents a ROC curve of the test, showing the specificity of differences. Wherein, A: samples of breast cancer and the control thereof, B: samples of cervical cancer and the control thereof, C: samples of esophageal cancer and the control thereof, D: samples of head and neck cancer and the control thereof, E: samples of lung adenocarcinoma and the control thereof, F: samples of pancreatic adenocarcinoma and the control thereof. It shows that average methylation values of SEQ ID NO. 14 target sequence in tumor samples of breast cancer, cervical cancer, head and neck cancer, esophageal cancer and lung adenocarcinoma were all higher than that in adjacent normal tissues. In tumor samples of pancreatic adenocarcinoma, average methylation value of SEQ ID NO. 14 target sequence was mostly higher than that in adjacent normal tissues, with the mean also higher than that in adjacent normal tissues. According to average methylation values of SEQ ID NO. 14 target sequence in samples, it can be accurately distinguished in vast majority of the tumor samples from adjacent normal tissues.

The specific examples of the present disclosure have been described above in detail, but they are only used as examples. The present disclosure is not limited to the specific examples described above. For those skilled in the art, any equivalent modifications and substitutions to the present disclosure are also within the scope of the present disclosure. Therefore, these equivalent changes and modifications without departing from the spirit and scope of the present disclosure all fall within the scope of the present disclosure.

## Claims

1. A tumor marker, wherein,
the tumor marker is located on the chromosome 1, chromosome 2, chromosome 3, chromosome 5, chromosome 7, chromosome 8, chromosome 10, chromosome 11, and chromosome 21 of the human genome, and comprises one or more CpG sites can be subject to methylation modification.

2. The tumor marker according to claim 1, wherein,
the tumor marker comprises one or more regions corresponding to the human genome hg19 as the reference version located at
chr2:105459135-105459190, chr10:124902392-124902455,
chr3:157812331-157812498, chr21:38378275-38378539,
chr8:97170353-97170404, chr5:134880362-134880455,
chr10:94835119-94835252, chr11:31826557-31826963,
chr3:147114032-147114108, chr10:50819227-50819589,
chr2:66809255-66809281, chr7:97361393-97361461,
chr8:70984200-70984294, chr1:6515341-6515409.

3. The tumor marker according to claim 1, wherein,
the methylation modification comprises 5-formylcytosine(5fC) modification, 5-hydroxymethylcytosine(5hmC) modification, 5-methylcytosine(5mC) modification or 5-carboxylcytosine(5-caC) modification.

4. The tumor marker according to claim 3, wherein,
the sequence of chr2:105459135-105459190 region is selected from the following group:
a) a base sequence as shown in SEQ ID NO.1;
b) a complementary sequence of the base sequence as shown in SEQ ID NO.1;
c) a nucleotide sequence or a complementary sequence thereof with at least 70% homology to SEQ ID NO.1.
and/or the sequence of chr10:124902392-124902455 region is selected from the following group:
a) a base sequence as shown in SEQ ID NO.2;
b) a complementary sequence of the base sequence as shown in SEQ ID NO.2;
c) a nucleotide sequence or a complementary sequence thereof with at least 70% homology to SEQ ID NO.2.
and/or the sequence of chr3: 157812331-157812498 region is selected from the following group:
a) a base sequence as shown in SEQ ID NO.3;
b) a complementary sequence of the base sequence as shown in SEQ ID NO.3;
c) a nucleotide sequence or a complementary sequence thereof with at least 70% homology to SEQ ID NO.3.
and/or the sequence of chr21:38378275-38378539 region is selected from the following group:
a) a base sequence as shown in SEQ ID NO.4;
b) a complementary sequence of the base sequence as shown in SEQ ID NO.4;
c) a nucleotide sequence or a complementary sequence thereof with at least 70% homology to SEQ ID NO.4.
and/or the sequence of chr8:97170353-97170404 region is selected from the following group:
a) a base sequence as shown in SEQ ID NO.5;
b) a complementary sequence of the base sequence as shown in SEQ ID NO.5;
c) a nucleotide sequence or a complementary sequence thereof with at least 70% homology to SEQ ID NO.5.
and/or the sequence of chr5: 134880362-134880455 region is selected from the following group:
a) a base sequence as shown in SEQ ID NO.6;
b) a complementary sequence of the base sequence as shown in SEQ ID NO.6;
c) a nucleotide sequence or a complementary sequence thereof with at least 70% homology to SEQ ID NO.6.
and/or the sequence of chr10:94835119-94835252 region is selected from the following group:
a) a base sequence as shown in SEQ ID NO.7;
b) a complementary sequence of the base sequence as shown in SEQ ID NO.7;
c) a nucleotide sequence or a complementary sequence thereof with at least 70% homology to SEQ ID NO.7.
and/or the sequence of chr11:31826557-31826963 region is selected from the following group:
a) a base sequence as shown in SEQ ID NO.8;
b) a complementary sequence of the base sequence as shown in SEQ ID NO.8;
c) a nucleotide sequence or a complementary sequence thereof with at least 70% homology to SEQ ID NO.8.
and/or the chr3:147114032-147114108 region is selected from the following group:
a) a base sequence as shown in SEQ ID NO.9;
b) a complementary sequence of the base sequence as shown in SEQ ID NO.9;
c) a nucleotide sequence or a complementary sequence thereof with at least 70% homology to SEQ ID NO.9.
and/or the sequence of chr10:50819227-50819589 region is selected from the following group:
a) a base sequence as shown in SEQ ID NO.10;
b) a complementary sequence of the base sequence as shown in SEQ ID NO.10;
c) a nucleotide sequence or a complementary sequence thereof with at least 70% homology to SEQ ID NO.10.
and/or the sequence of chr2:66809255-66809281 region is selected from the following group:
a) a base sequence as shown in SEQ ID NO.11;
b) a complementary sequence of the base sequence as shown in SEQ ID NO.11;
c) a nucleotide sequence or a complementary sequence thereof with at least 70% homology to SEQ ID NO.11.
and/or the sequence of chr7:97361393-97361461 region is selected from the following group:
a) a base sequence as shown in SEQ ID NO.12;
b) a complementary sequence of the base sequence as shown in SEQ ID NO.12;
c) a nucleotide sequence or a complementary sequence thereof with at least 70% homology to SEQ ID NO.12.
and/or the sequence of chr8:70984200-70984294 region is selected from the following group:
a) a base sequence as shown in SEQ ID NO.13;
b) a complementary sequence of the base sequence as shown in SEQ ID NO.13;
c) a nucleotide sequence or a complementary sequence thereof with at least 70% homology to SEQ ID NO.13.
and/or the sequence of chr1:6515341-6515409 region is selected from the following group:
a) a base sequence as shown in SEQ ID NO.14;
b) a complementary sequence of the base sequence as shown in SEQ ID NO.14;
c) a nucleotide sequence or a complementary sequence thereof with at least 70% homology to SEQ ID NO.14.

5. A use of the tumor marker according to any one of claims 1 to 4 in tumor screening, prognosis, diagnostic reagents and drug targets.

6. The use according to claim 5, wherein,
it comprises detecting the methylation status of CpG sites of the tumor markers.

7. The application according to claim 5, wherein,
the tumor comprises: BLCA, CESC, CHOL, COADREAD, ESCA, GBM, HNSC, KIRC, KIRP, LIHC, LUNG, PAAD, PCPG, PRAD, SARC, SKCM, STAD, THCA, THYM, UCEC, BC.

8. The application according to claim 7, wherein,
the tumor comprises: BC, CESC, ESCA, HNSC, LUNG, PAAD.

9. A drug used for inhibiting tumor proliferation, wherein,
the drug comprises an inhibitor of the tumor markers according to any one of the claims 1 to 4.

10. The drug according to claim 9, wherein,
the inhibitor is a methylation inhibitor.

11. The drug according to claim 9, wherein,
it further comprises a pharmaceutically acceptable carrier.

12. A method for detecting tumor markers, wherein,
comprising the following steps:
S1. Obtaining a tissue sample to be tested;
S2. Extracting DNA of the tissue sample to be tested, and obtaining a methylation value of the sample;
S3. Calculating methylation status of each CpG site in the sequence regions of tumor markers according to any one of the claims 1 to 4 or the average methylation status of the entire region.

13. The method according to claim 10, wherein,
the method for obtaining the methylation value of the sample in step S2 comprises: sequencing, probes detecting, antibodies detecting, and mass spectrometry detecting.

14. A kit for detecting tumor markers, wherein,
it comprises primers or probes for specifically detecting the tumor markers according to any one of the claims 1 to 4.

15. The kit for detecting tumor markers according to claim 14, wherein,
said specifically detecting of the tumor markers comprises specific ally detecting of the methylation of CpG sites of the tumor markers.

16. The kit for detecting tumor markers according to claim 15, wherein,
the methylation of CpG sites of tumor markers comprises 5-formylcytosine(5fC) modification, 5-hydroxymethylcytosine(5hmC) modification, 5-methylcytosine(5mC) modification or 5-carboxylcytosine(5-caC) modification.
